# EUROPEAN PATENT APPLICATION

(11) **EP 4 541 797 A1**
(43) Date of publication of application: **23.04.2025**
(21) Application number: 23845379.9
(22) Date of filing: 18.07.2023
(51) Int. Cl.: C07D 491/22, C07D 519/00, C07D 498/22, A61P 35/00, A61K 31/5365, A61K 31/496, A61K 31/4745, A61K 31/5377, A61K 47/68

(54) **CAMPTOTHECIN DERIVATIVE, METHOD FOR PREPARING SAME, AND USE THEREOF**

(30) Priority: 29.07.2022 CN 202210913611
(71) Applicant: Hangzhou Adcoris Biopharma Co., Ltd, Hangzhou, Zhejiang 310056 (CN)
(72) Inventor: HUANG, Yunsheng, Hangzhou, Zhejiang 311258 (CN); LI, Yaowu, Hangzhou, Zhejiang 311258 (CN); ZHANG, Shiyi, Hangzhou, Zhejiang 311258 (CN)
(74) Representative: Elzaburu S.L.P.
(86) International application number: PCT/CN2023/107935
(87) International publication number: WO 2024/022167

(57) **Abstract**

The present invention provides a novel camptothecin derivative compound, a pharmaceutically acceptable salt, a stereoisomer, or a prodrug thereof, and a method for preparing same and use thereof. The compound has a structure represented by formula (I) and has good anticancer activity.

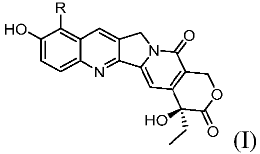

## Description

### TECHNICAL FIELD

The present disclosure relates to the field of medicine, and specifically to a series of structurally novel camptothecin derivatives, and preparation methods and use thereof.

### BACKGROUND

Camptothecin (CPT) is a natural product originally extracted by American scientists Wall and Wani from the Chinese plant, *Camptotheca acuminata.* Camptothecin is a water-insoluble, cytotoxic quinoline alkaloid with broad-spectrum antitumor activities. Its mechanism of action is considered to involve binding to topoisomerase Top1 and DNA to form a complex, inhibiting Top1 activity, blocking DNA replication and protein synthesis, thereby leading to apoptosis. Camptothecin shows significant efficacy against gastric cancer, esophageal cancer, cardia cancer, colon cancer, rectal cancer, primary liver cancer, acute and chronic granulocytic leukemia, choroidal epithelial cancer, lung cancer, bladder cancer, etc. Despite its excellent antitumor activity, so far, only two camptothecin-based compounds have been developed globally as anticancer drugs, Irinotecan, which is used for intestinal cancer and small cell lung cancer (SCLC), and Topotecan, which is used for lung cancer and ovarian cancer. Another camptothecin derivative, Belotecan, is approved only in the Republic of Korea for treating SCLC and ovarian cancer. Camptothecin, due to its cytotoxicity, poor solubility, and drug resistance, has been prevented from its broader application in cancer treatment, and drug resistance thereto has been developed.

Therefore, the development of camptothecin derivative drugs with low toxicity, with water solubility, and with resistance to the drug resistance is necessary and has broad application value.

### SUMMARY OF THE INVENTION

The purpose of the present disclosure is to provide a structurally novel camptothecin derivative with anticancer activity.

In one aspect, the present disclosure provides a compound represented by formula (I) or a pharmaceutically acceptable salt, a stereoisomer, or a prodrug thereof: 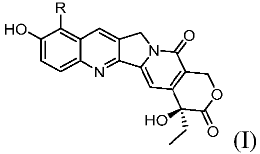 wherein,
R is -CH₂NR₁R₂, wherein, R₁ and R₂ are each independently selected from hydrogen, hydroxyl, C₁-C₆ alkyl, C₁-C₆ alkoxy, C₃-C₆ cycloalkyl, 4 to 8 membered heterocycloalkyl, 4 to 8 membered aryl or -NRₐR_{b}, the C₁-C₆ alkyl, C₁-C₆ alkoxy, C₃-C₆ cycloalkyl, 4 to 8 membered heterocycloalkyl, 4 to 8 membered aryl or -NRₐR_{b} is optionally substituted with substituents selected from halogen, hydroxyl, amino, carboxyl, C₁-C₆ alkoxy, C₁-C₆ alkoxycarbonyl, -O-(C₁-C₃ alkyl)-NRₐR_{b} or 4 to 8 membered heterocycloalkyl, provided that R₁, R₂ are not both hydrogen or methyl; or,
R₁, R₂ and the N atom to which they connect collectively form a 4 to 8 membered cycloalkyl, 4 to 8 membered heterocycloalkyl, 4 to 8 membered aryl or 9 to 12 membered azaspiroalkyl, the 4 to 8 membered cycloalkyl, 4 to 8 membered heterocycloalkyl, 4 to 8 membered aryl or 9 to 12 membered azaspiroalkyl is optionally substituted with substituents selected from hydroxyl, amino, carboxyl, C₁-C₆ alkoxy, C₁-C₆ alkoxycarbonyl, C₁-C₃ alkyl, C₁-C₃ alkoxy, -O-NH₂, -NRₐR_{b} or - C(O)R_{c}; or,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from C₁-C₆ alkyl, C₁-C₆ alkoxy, C₃-C₆ cycloalkyl, 4 to 8 membered heterocycloalkyl, -NRₐR_{b} or 4 to 8 membered heterocycloalkyl, the C₁-C₆ alkyl, C₁-C₆ alkoxy, C₃-C₆ cycloalkyl, 4 to 8 membered heterocycloalkyl, -NRₐR_{b} or 4 to 8 membered heterocycloalkyl is optionally substituted with substituents selected from halogen, hydroxyl, amino, carboxyl, C₁-C₆ alkoxy or C₁-C₆ alkoxycarbonyl, provided that at least one of R₁, R₂ is H.

Rₐ and R_{b} are each independently selected from H, C₁-C₆ alkyl or C₁-C₆ alkoxycarbonyl.

R_{c} is selected from amino, C₁-C₃ alkyl, C₁-C₃ alkoxy, C₃-C₆ cycloalkyl or hydroxyl-substituted C₁-C₃ alkyl.

In one embodiment, in the compound represented by formula (I), R is -CH₂NR₁R₂, wherein, R₁ and R₂ are each independently selected from hydrogen, hydroxyl, C₁-C₆ alkyl, C₁-C₆ alkoxy, C₃-C₆ cycloalkyl, 4 to 8 membered heterocycloalkyl or -NRₐR_{b}; the C₁-C₆ alkyl is optionally substituted with substituents selected from halogen, hydroxyl, amino, carboxyl, C₁-C₆ alkoxycarbonyl, -O-(C₁-C₃ alkyl)-NRₐR_{b} or 4 to 8 membered heterocycloalkyl; wherein, the C₃-C₆ cycloalkyl or 4 to 8 membered heterocycloalkyl is optionally substituted with substituents selected from hydroxyl, C₁-C₃ alkoxy or C₁-C₆ alkoxycarbonyl, provided that R₁, R₂ are not both hydrogen or methyl; or,
R₁, R₂ and the N atom to which they connect collectively form a 4 to 8 membered heterocycloalkyl or 9 to 12 membered azaspiroalkyl, the 4 to 8 membered heterocycloalkyl is optionally substituted with substituents selected from hydroxyl, amino, carboxyl, C₁-C₆ alkoxycarbonyl, C₁-C₃ alkyl, C₁-C₃ alkoxy, -O-NH₂ or -C(O)R_{c}; or,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from halogen-substituted C₁-C₆ alkyl, C₃-C₆ cycloalkyl or 4 to 8 membered heterocycloalkyl, provided that at least one of R₁, R₂ is H.

In one embodiment, Rₐ and R_{b} are each independently selected from H, methyl or tert-butoxycarbonyl.

In one embodiment, R_{c} is selected from amino, C₁-C₃ alkyl, C₁-C₃ alkoxy, C₃-C₆ cycloalkyl or hydroxyl-substituted C₁-C₃ alkyl.

In one embodiment, in the compound represented by formula (I), R is -CH₂NHR₁, R₁ is selected from hydrogen, hydroxyl, C₁-C₆ alkyl, C₁-C₆ alkoxy, C₃-C₆ cycloalkyl, 4 to 8 membered heterocycloalkyl or -NRₐR_{b}, the C₁-C₆ alkyl is optionally substituted with substituents selected from halogen, hydroxyl, amino, carboxyl, C₁-C₆ alkoxycarbonyl, -O-(C₁-C₃ alkyl)-NRₐR_{b} or 4 to 8 membered heterocycloalkyl, the C₃-C₆ cycloalkyl or 4 to 8 membered heterocycloalkyl is optionally substituted with substituents selected from hydroxyl, C₁-C₃ alkoxy or C₁-C₆ alkoxycarbonyl, provided that, R₁ is not hydrogen; or,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from halogen-substituted C₁-C₆ alkyl, C₃-C₆ cycloalkyl or 4 to 8 membered heterocycloalkyl.

In one embodiment, Rₐ and R_{b} are each independently selected from H, methyl or tert-butoxycarbonyl.

In one embodiment, R₁ and R₂ are each independently selected from hydrogen, hydroxyl, methyl, ethyl, propyl, isopropyl, methoxy, ethoxy, propoxy, cyclopropyl, cyclopentyl, cyclohexanyl, 5 or 6-membered heterocycloalkyl containing 1 to 2 nitrogen atoms as ring atom, 5 or 6-membered heterocycloalkyl containing 1 to 3 heteroatoms selected from N or O as ring atom or -NRₐR_{b}, wherein, the methyl, ethyl, propyl or isopropyl is optionally substituted with substituents selected from halogen, hydroxyl, amino, carboxyl, methoxycarbonyl, ethoxycarbonyl, tert-butoxycarbonyl, methoxy-NRₐR_{b}, ethoxy-NRₐR_{b}, pyrrolidinyl, piperidinyl or piperazinyl; the cyclopropyl, cyclopentyl, cyclohexanyl, 5 or 6-membered heterocycloalkyl containing 1 to 2 nitrogen atoms as ring atom or 5 or 6-membered heterocycloalkyl containing 1 to 3 heteroatoms selected from N or O as ring atom is optionally substituted with substituents selected from hydroxyl, methoxy, ethoxy, propoxy or methoxycarbonyl, ethoxycarbonyl or tert-butoxycarbonyl, provided that, R₁, R₂ are not both hydrogen or methyl.

In one embodiment, R₁, R₂ and the N atom to which they connect collectively form pyrrolidinyl, piperidinyl, piperazinyl or 11 membered azaspiroalkyl, the pyrrolidinyl, piperidinyl or piperazinyl or 11 membered azaspiroalkyl is optionally substituted with substituents selected from hydroxyl, amino, carboxyl, methoxycarbonyl, ethoxycarbonyl, tert-butoxycarbonyl, methyl, ethyl, propyl, isopropyl, methoxy, ethoxy, propoxy, -O-NH₂ or -C(O)R_{c}.

In one embodiment, R, with the adjacent hydroxyl and the C atom on the benzene ring to which it connects collectively form pyrrolidinyl, piperidinyl or piperazinyl or oxazinyl, the pyrrolidinyl, piperidinyl, piperazinyl or oxazinyl is optionally substituted with substituents selected from methyl, ethyl, propyl, isopropyl, cyclopropyl, cyclopentyl, cyclohexanyl, pyrrolidinyl, piperidinyl, piperazinyl or oxazinyl.

In one embodiment, in the compound represented by formula (I), R₁ and R₂ are each independently selected from hydrogen, hydroxyl, amino, methyl, ethyl, isopropyl, methoxy, ethoxy, trifluoroethyl, cyclopropyl, -(CH₂)₂OH, -(CH₂)₂-₄NH₂, -CH(CH₃)COOH, - CH(CH₃)CH₂OCH₃, -CH(CH₃)CH₂OH, -(CH₂)₂CH₃, -NHBoc, N(CH₃)Boc, -CH(CH₃)Boc, - (CH₂)₂O(CH₂)₂NHBoc, 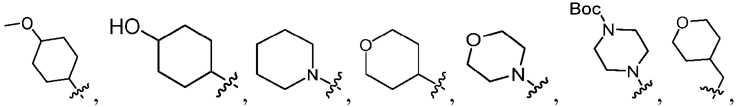 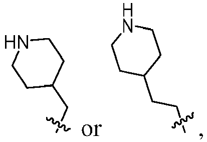 provided that, R₁, R₂ are not both hydrogen or methyl; or,
R₁, R₂ and the N atom to which they connect collectively form pyrrolidine, piperidine, piperazine or 3,9-diazaspiroundecane (for example 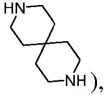 the pyrrolidine, piperidine, piperazine or 3,9-diazaspiroundecane is optionally substituted with substituents selected from hydroxyl, amino, methyl, methoxy, carboxyl, tert-butoxycarbonyl, -O-NH₂ or -C(O)R_{c}; or,
R, with the adjacent hydroxyl and the C atom on the benzene ring to which it connects collectively form 1,3-oxazinane, the 1,3-oxazinane is optionally substituted with substituents selected from trifluoromethyl, cyclopropyl or morpholine-4-yl, provided that at least one of R₁, R₂ is H.

R_{c} is selected from amino, methyl, methoxy, hydroxyethyl or cyclopropyl.

In one embodiment, R, with the adjacent hydroxyl and the C atom on the benzene ring to which it connects collectively form 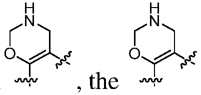 is optionally substituted with substituents selected from C₁-C₆ alkyl or 4 to 8 membered heterocycloalkyl.

In one embodiment, R, with the adjacent hydroxyl and the C atom on the benzene ring to which it connects collectively form 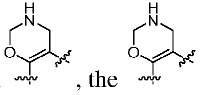 is optionally substituted with substituents selected from trifluoromethyl, cyclopropyl or morpholine-4-yl, provided that at least one of R₁, R₂ is H.

In one embodiment, R₁, R₂ and the N atom to which they connect collectively form a pyrrolidine, piperidine, piperazine or 3,9-diazaspiroundecane, the pyrrolidine, piperidine, piperazine or 3,9-diazaspiroundecane is optionally substituted with substituents selected from amino, methoxy, -O-NH₂, carboxyl, tert-butoxycarbonyl, -C(O)-NH₂, -C(O)OCH₃, -C(O)CH₃, - C(O)-cyclopropyl, -C(O)CH₂OH.

In one embodiment, R is-CH₂NHR₁, R₁ is selected from hydrogen, hydroxyl, amino, methyl, ethyl, isopropyl, methoxy, ethoxy, trifluoroethyl, cyclopropyl, -(CH₂)₂OH, -(CH₂)₂-₄NH₂, - CH(CH₃)COOH, -CH(CH₃)CH₂OCH₃, -CH(CH₃)CH₂OH, -(CH₂)₂CH₃, -NHBoc, N(CH₃)Boc, - CH(CH₃)Boc, -(CH₂)₂O(CH₂)₂NHBoc, 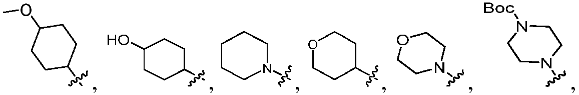 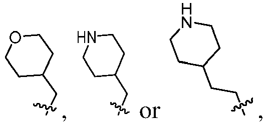 provided that, R₁ is not hydrogen; or,
R, with the adjacent hydroxyl and the C atom on the benzene ring to which it connects collectively form 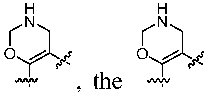 is optionally substituted with substituents selected from trifluoromethyl, cyclopropyl or morpholine-4-yl.

In one embodiment, R, with the adjacent hydroxyl and the C atom on the benzene ring to which it connects collectively form 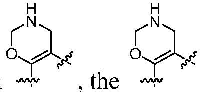 is optionally substituted with substituents selected from trifluoromethyl or morpholine-4-yl.

In one embodiment, R is selected from the following structure:

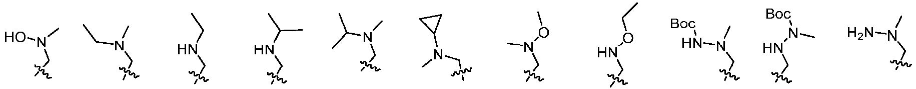

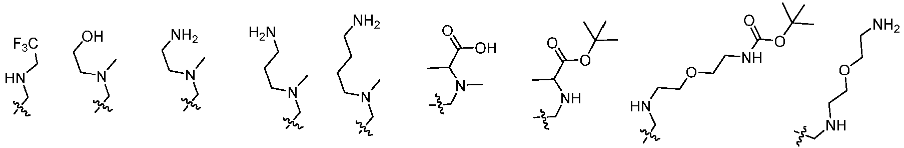

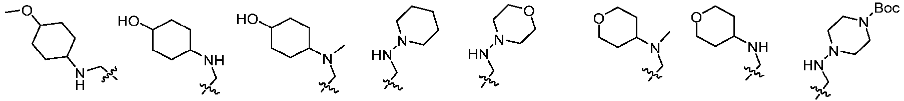

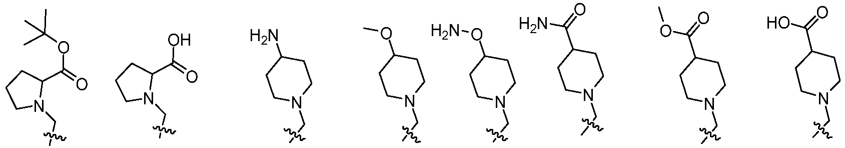

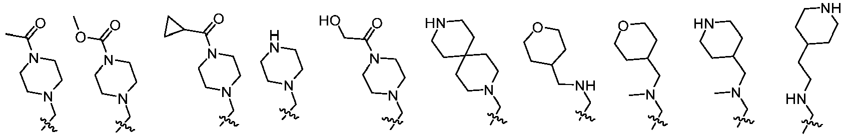

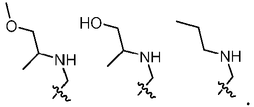

In one embodiment, R is selected from the following structure:

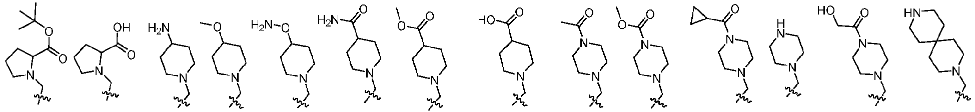

In one embodiment, R is selected from the following structure:

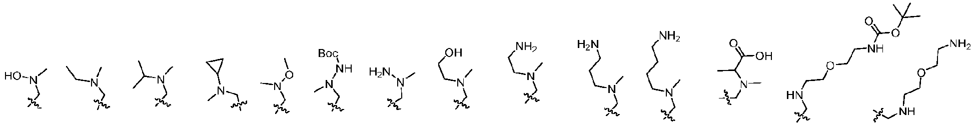

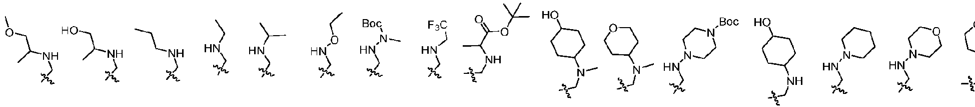

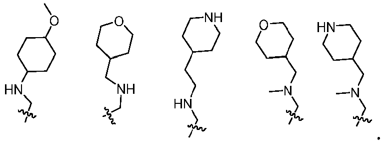

In the present disclosure, R in the above compounds does not include the following structure: -CH₂NHCH₂CH₂OH, -CH₂NHCH₂CH₂N(CH₃)₂, 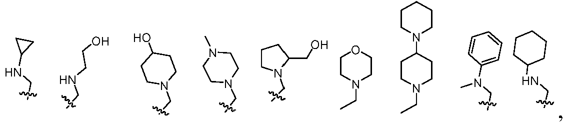 and, the compound represented by formula (I) exclude the following compound:

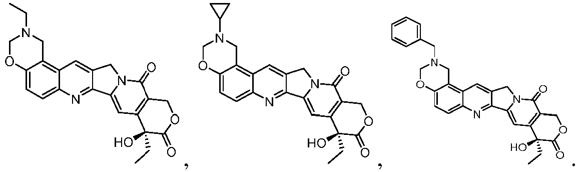

In one embodiment, the compound represented by fomula (I) is the compound represented by formula (II): 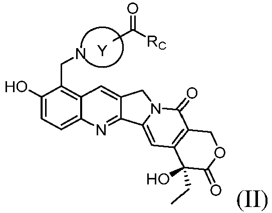 wherein,
ring Y is selected from 4 to 8 membered nitrogen-containing saturated ring, 4 to 8 membered nitrogen-containing aromatic ring or 9 to 12 membered nitrogen-containing saturated spiral ring, the 4 to 8 membered saturated nitrogen-containing ring, 4 to 8 membered nitrogen-containing aromatic ring or 9 to 12 membered nitrogen-containing saturated spiral ring is optionally further substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl.
preferably, ring Y is selected from 4 to 8 membered saturated nitrogen-containing ring or 9 to 12 membered nitrogen-containing saturated spiral ring, the 4 to 8 membered saturated nitrogen-containing ring is optionally substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl.
R_{c} is selected from hydroxyl, amino, C₁-C₃ alkyl, C₁-C₃ alkoxy, C₃-C₆ cycloalkyl or hydroxyl-substituted C₁-C₃ alkyl.
preferably, Rc is selected from hydroxyl, amino, methyl, methoxy, hydroxymethyl, tert-butoxy or cyclopropyl.

In one embodiment, the compound represented by fomula (II) is the compound represented by formula (IIa), formula (IIb) or formula (IIc): 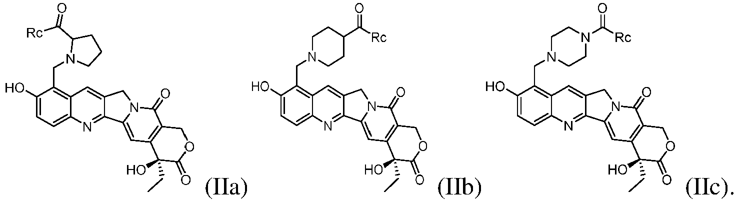
wherein, R_{c} is selected from hydroxyl, amino, C₁-C₃ alkyl, C₁-C₃ alkoxy, C₃-C₆ cycloalkyl or hydroxyl-substituted C₁-C₃ alkyl.
preferably, R_{c} is selected from hydroxyl, amino, methyl, methoxy, hydroxymethyl, tert-butoxy or cyclopropyl. 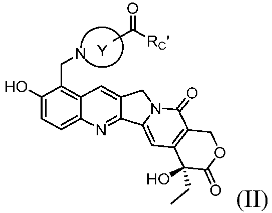 wherein,
   ring Y is selected from 4 to 8 membered nitrogen-containing saturated ring, 4 to 8 membered nitrogen-containing aromatic ring or 9 to 12 membered nitrogen-containing saturated spiral ring, the 4 to 8 membered saturated nitrogen-containing ring, 4 to 8 membered nitrogen-containing aromatic ring or 9 to 12 membered nitrogen-containing saturated spiral ring is optionally further substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl.
   wherein, R_{c} is defined the same as the compound represented by formula (I).

Preferably, ring Y is selected from 4 to 8 membered saturated nitrogen-containing ring or 9 to 12 membered nitrogen-containing saturated spiral ring, the 4 to 8 membered saturated nitrogen-containing ring is optionally substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl.

R_{c}' is selected from hydroxyl, amino, C₁-C₃ alkyl, C₁-C₃ alkoxy, C₃-C₆ cycloalkyl or hydroxyl-substituted C₁-C₃ alkyl.

Preferably, R_{c}' is selected from hydroxyl, amino, methyl, methoxy, hydroxymethyl, tert-butoxy or cyclopropyl.

In one embodiment, the compound represented by fomula (II) is the compound represented by formula (IIa), formula (IIb) or formula (IIc): 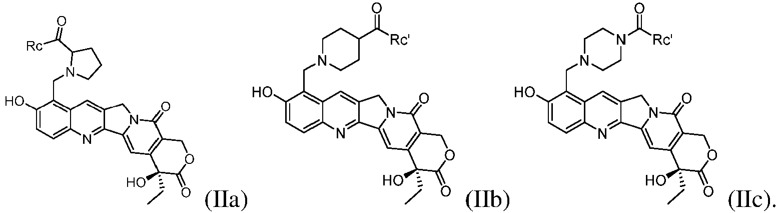
wherein, R_{c}' is selected from hydroxyl, amino, C₁-C₃ alkyl, C₁-C₃ alkoxy, C₃-C₆ cycloalkyl or hydroxyl-substituted C₁-C₃ alkyl.
preferably, R_{c}' is selected from hydroxyl, amino, methyl, methoxy, hydroxymethyl, tert-butoxy or cyclopropyl.

The present disclosure provides the following compound:

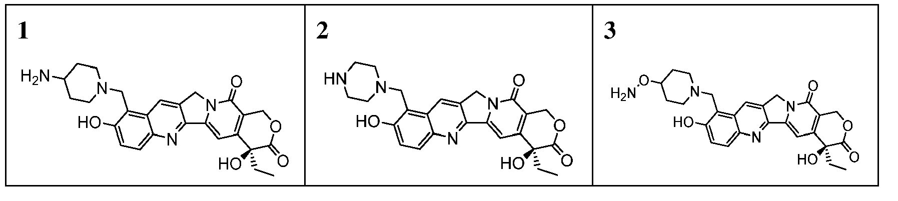

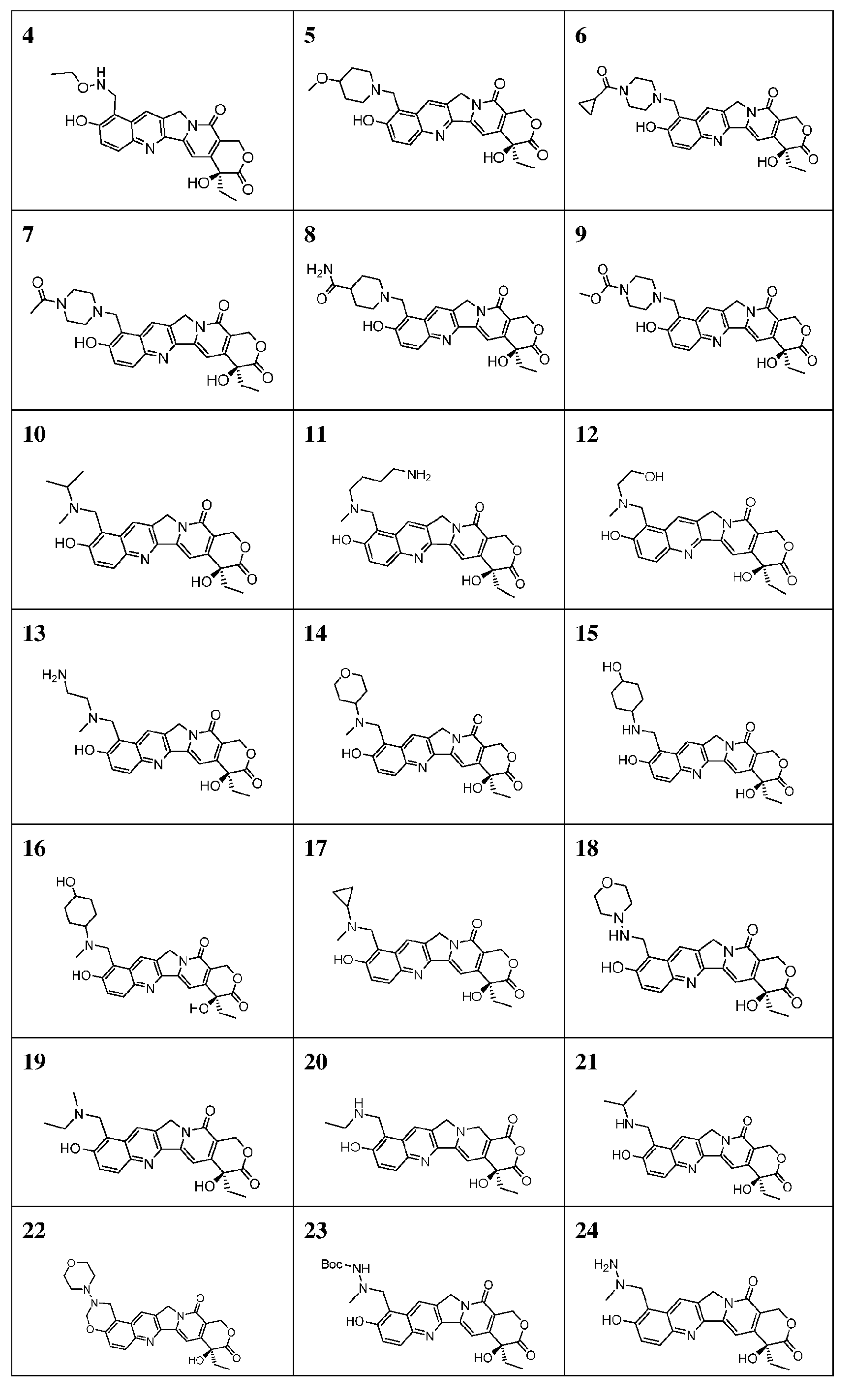

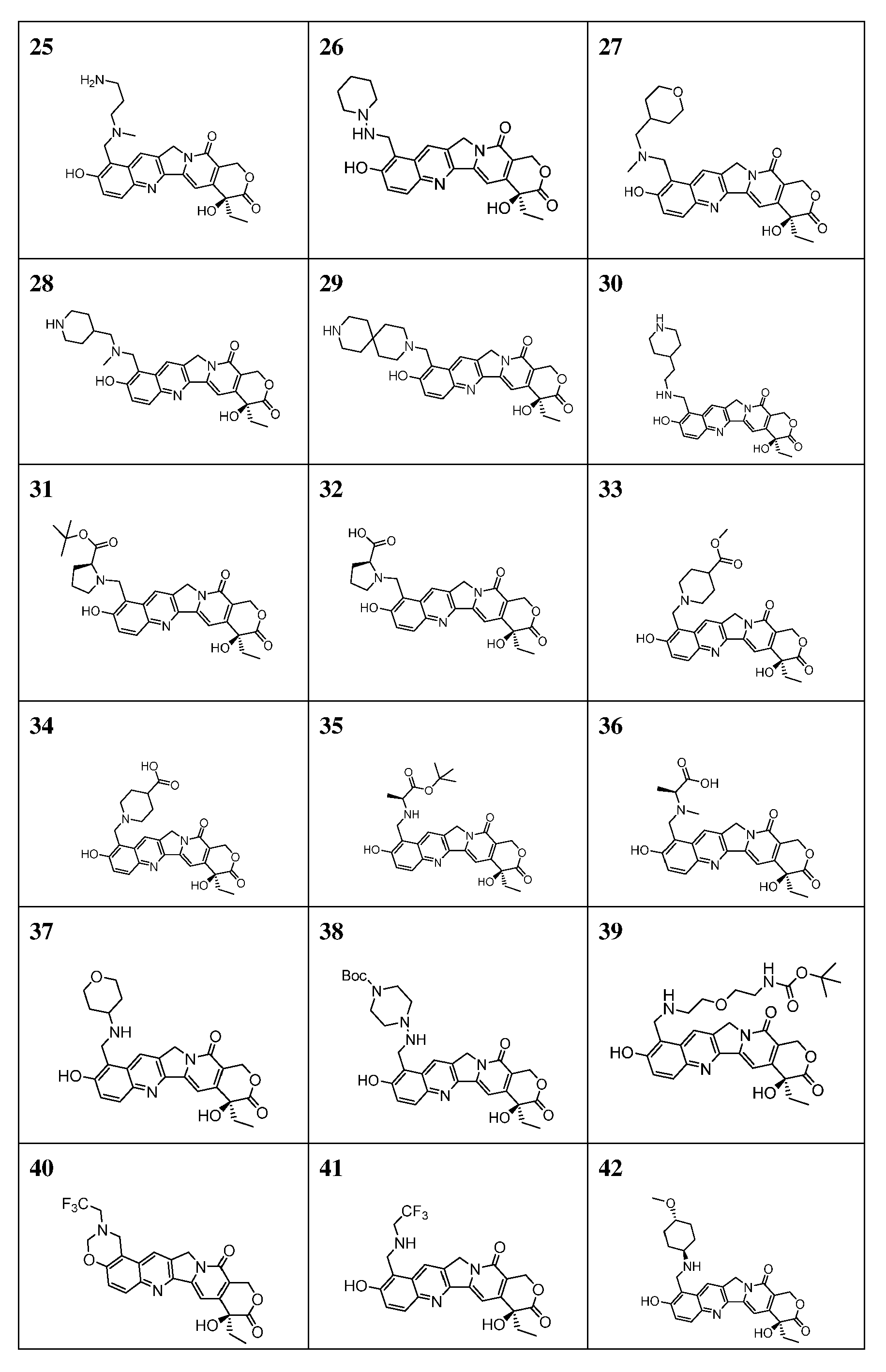

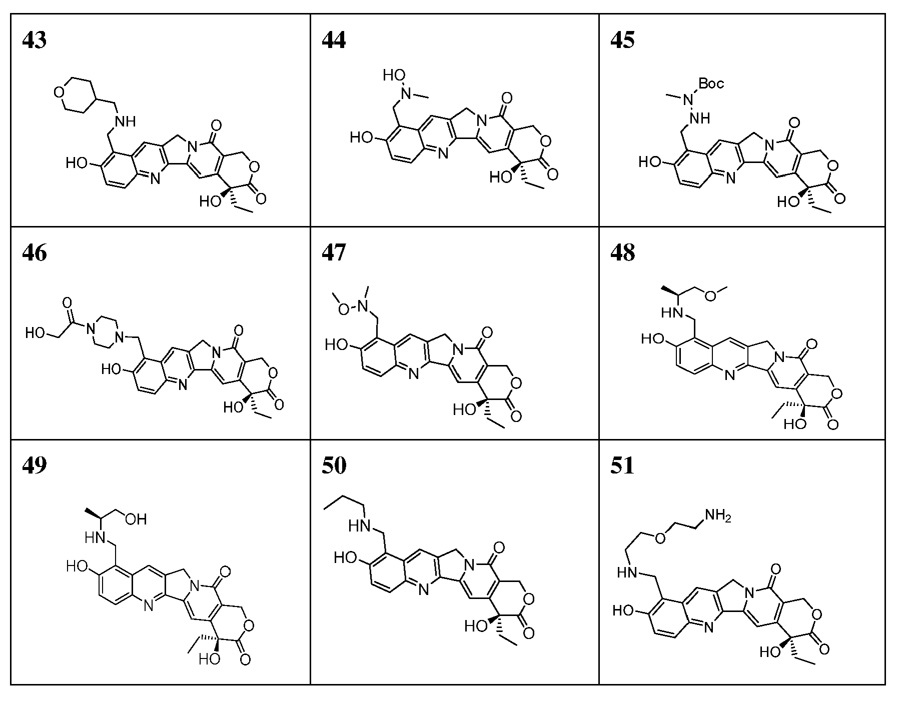

In another aspect, the present disclosure provides a method for preparing the above compound comprising steps selected the following reaction scheme: 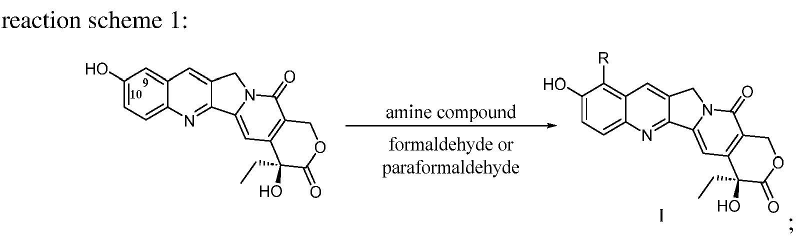
9-position Mannich reaction of 10-hydroxycamptothecin, formaldehyde or paraformaldehyde, and amine compound is carried out to afford the compound represented by formula (I);
wherein, the amine compound is NHR₁R₂, and R, R₁ and R₂ are each defined the same as above. 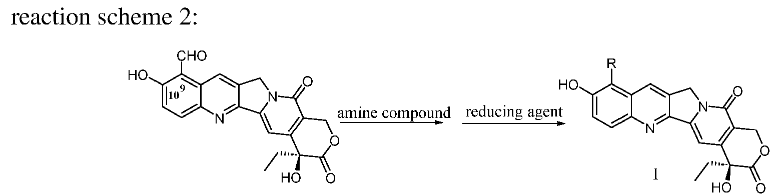
   9-formyl-10-hydroxycamptothecin, amine coumpound and sodium cyanoborohydride are subjected to reductive amination reaction to afford the compound represented by formula (I);
   wherein, the amine compound is NHR₁R₂, and R, R₁ and R₂ are each defined the same as above.

In one embodiment, the 9-formyl-10-hydroxycamptothecin is afforded by the reaction of 10-hydroxycamptothecin and hexamethylenetetramine.

In one embodiment, in the Mannich reaction according to reaction scheme 1, the amount of paraformaldehyde or formaldehyde is 1.0 to 5.0 molar equivalents; preferably, is 1.0 to 3.0 molar equivalents; more preferably, is 1.2 to 2.2 molar equivalents.
preferably, in reaction scheme 1, the amount of amine compound is 1.0 to 5.0 molar equivalents; preferably, is 1.0 to 2.0 molar equivalents; more preferably, is 1.2 to 1.6 molar equivalents.

In one embodiment, in the reductive amination reaction according to reaction scheme 2, the reducing agent is sodium cyanoborohydride.
preferably, in reaction scheme 2, the amount of amine compound is 1.0 to 3.0 molar equivalents; more preferably, is 1.0 to 1.2 molar equivalents.
preferably, the amount of sodium cyanoborohydride is 1.0 to 3.0 molar equivalents; more preferably, is 1.0 to 2.0 molar equivalents; more preferably, is 1.2 to 1.5 molar equivalents.

In another aspect, the present disclosure provides an antibody drug conjugate comprising a small molecule drug, a linker and a antibody, wherein the above compound or pharmaceutically acceptable salt, stereoisomer or prodrug thereof is the small molecule drug.

In another aspect, the present disclosure provides a pharmaceutical composition comprising the above compound or pharmaceutically acceptable salt, stereoisomer, or prodrug thereof, or the above antibody-drug conjugate.

The pharmaceutical composition further comprises pharmaceutically acceptable excipients.

In another aspect, the present disclosure provides a use of the above compound or pharmaceutically acceptable salt, stereoisomer, prodrug thereof, the antibody-drug conjugate, or the pharmaceutical composition in the manufacture of a medicament for treating a cancer.

In one embodiment, the cancer includes gastric cancer, esophageal cancer, cardia cancer, breast cancer, ovarian cancer, colon cancer, rectal cancer, primary liver cancer, acute and chronic granulocytic leukemia, choroidal epithelial cancer, lung cancer, bladder cancer, intestinal cancer and small cell lung cancer; more preferably, the cancers are esophageal cancer, breast cancer and gastric cancer.

In another aspect, the present disclosure provides a method of treating a cancer comprising the step of administering to a patient in need thereof the above compound or pharmaceutically acceptable salt, stereoisomer, prodrug thereof, the antibody-drug conjugate, or the pharmaceutical composition.

In one embodiment, the above compound or pharmaceutically acceptable salt, stereoisomer, prodrug, antibody drug conjugate or pharmaceutical composition thereof are administered in therapeutically effective amounts.

The camptothecin derivative compound provided by the present disclosure has obvious inhibitory activity on cancer cells such as esophageal cancer cell OE33, breast adenocarcinoma cell SK-BR-3, gastric cancer cell NCI-N87, etc.

Notably, the present disclosure also provides the method for preparing the above compounds, which have surprisingly higher yields.

### DETAILED DESCRIPTION OF THE EMBODIMENTS

### I. defination

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be open-ended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclusure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

The compound of the present disclosure also includes tautomeric forms. Tautomeric forms arise from the exchange of one single bond with an adjacent double bond and together with the transfer of one proton.

A numerical range as used herein refers to each integer within the given range. For example,"C1-C6" refers to the group that can have 1 carbon atom, 2 carbon atoms, 3 carbon atoms, 4 carbon atoms, 5 carbon atoms or 6 carbon atoms; "C3-C6" refers to the group that can have 3 carbon atoms, 4 carbon atoms, 5 carbon atoms or 6 carbon atoms.

The term "substituted" refers to the replacement of any one or more hydrogen atoms on a specific atom or group with a substituent, provided that the valency of the specific atom or group is normal and the resulting compound is stable. Unless otherwise specified, the type and number of substituents can be arbitrary, based on what is chemically feasible.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "alkyl" refers to saturated aliphatic hydrocarbon groups, which is a straight or branched chain group containing 1 to 20 carbon atoms, preferably the alkyl containing 1 to 8 carbon atoms, more preferably 1 to 6 carbon atoms, and most preferably 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, and various branched isomers thereof, etc., more preferably, a lower alkyl containing 1 to 6 carbon atoms, non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, substituents can be present at any available point of attachment, the substituents preferably being one or more of the following groups, independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group, with methyl, ethyl, isopropyl, tert-butyl, halogenated alkyl, deuterated alkyl, alkoxy-substituted alkyl, and hydroxy-substituted alkyl being preferred for the present disclosure.

The term "heterocyclyl" refers to a saturated or partially unsaturated monocyclic or multicyclic cyclic hydrocarbon substituent containing 3 to 20 ring atoms, wherein one or more ring atoms are heteroatoms selected from nitrogen, oxygen, or S(O)m (wherein m is an integer from 0 to 2), but excluding ring moieties of-O-O-, -O-S-, or-S-S-, the remaining ring atoms being carbon, preferably containing 3 to 12 ring atoms, of which 1 to 4 are heteroatoms; more preferably containing 3 to 8 ring atoms; most preferably containing 3 to 8 ring atoms; further preferably containing 3 to 8 membered heterocyclic with 1 to 3 nitrogen atoms, optionally substituted with 1 to 2 oxygen atoms, sulfur atoms, oxo, including nitrogen-containing monocyclic heterocyclic group, nitrogen-containing spiroheterocyclic group or nitrogen-containing fused heterocyclic group.

The term "heterocycloalkyl" refers to a saturated cyclic substituent with heteroatoms in the ring skeleton, wherein one or more (preferably 1 to 4, or 1 to 3, or 1 to 2) ring atoms are heteroatoms selected from N, O, S.

The term "aryl" refers to a 6 to 14 membered all-carbon monocyclic or fused polycyclic (rings sharing adjacent pairs of carbon atoms) group having a conjugated π electron system, preferably 6 to 12 membered, such as phenyl and naphthyl.

Aryl groups can be substituted or unsubstituted. When substituted, the substituents are preferably one or more of the following groups, independently selected from: alkyl, alkenyl, alkynyl, alkoxy, alkylthio, alkylamino, halogen, thio, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(unsubstituted cycloalkyl), wherein the definition of alkyl is as described above. Non-limiting examples of alkoxy include: methoxy, ethoxy, propoxy, butoxy, cyclopropoxy, cyclobutoxy, cyclopentoxy, cyclohexoxy. Alkoxy can be optionally substituted or unsubstituted, and when substituted, the substituents are preferably one or more of the following groups, independently selected from: alkyl, alkenyl, alkynyl, alkoxy, alkylthio, alkylamino, halogen, thio, hydroxy, nitro, chloro, cycloalkyl, heterocycloalkyl, aryl, heteroaryl, cycloalkoxy, heterocycloalkoxy, cycloalkylthio, heterocycloalkylthio, carboxyl, or carboxylate group.

The hydrogen atoms according to the present disclosure can be substituted by the isotope deuterium thereof.

The term "optional" or "optionally" refers to the subsequently described event or circumstance may but not necessarily occur, and the description includes instances where the event or circumstance occurs or does not occur. For example, "heterocyclic group optionally substituted with alkyl" means that the alkyl may but not necessarily exist, and the description includes both instances where the heterocyclic group is substituted with alkyl and instances where the heterocyclic group is not substituted with alkyl.

The term "substituted" refers to one or more hydrogen atoms in a group, preferably up to 5, and more preferably 1-3 hydrogen atoms, being independently substituted with the corresponding number of substituents. It goes without saying that the substituents are only in their possible chemical positions, and those skilled in the art can determine (through experiment or theory) which substitutions are possible or impossible without undue effort. For example, combinations of an amino or hydroxy having a free hydrogen with a carbon atom having an unsaturated (e.g., olefinic) bond may be unstable.

" 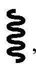 " refers to the chemical bond junction.

### Medicament or pharmaceutical composition

As used herein, "pharmaceutically acceptable salt" refers to the salt of the corresponding amine compound with an inorganic or organic acid, or the salt of the corresponding carboxyllic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids and so on.

As used herein, "prodrug" refers to the form in which a compound is metabolized or simply chemically altered in the body of a patient after the compound has been introduced into the human body in a suitable mode of administration to the compound contained in the general formula 1 of the present invention, as well as the corresponding salt. Prodrugs of the compounds include, but are not limited to, various carboxylate esters, carbonate esters, phosphate esters, sulfate esters, sulfonate esters, amino acid esters, gluconate esters, as well as various amides, acetals, hemiacetals, amidocarbonates and so on.

The drugs or pharmaceutical compositions of the present disclosure can be administered orally, topically, parenterally, or mucosally (e.g., buccally, by inhalation, or rectally) in dosage unit formulations comprising conventional non-toxic pharmaceutically acceptable carriers. It is often desirable to use orally. The active agent can be administered orally in the form of capsules, tablets, etc. (see Remington: The Science and Practice of Pharmacy, 20th Edition).

For oral administration in the form of tablets or capsules, the active pharmaceutical ingredients can be combined with non-toxic, pharmaceutically acceptable excipients such as binders (for example, pregelatinized corn starch, polyvinyl pyrrolidone, or hydroxypropyl methylcellulose); fillers (for example, lactose, sucrose, glucose, mannitol, sorbitol, and other reducing and non-reducing sugars, microcrystalline cellulose, calcium sulfate, or calcium hydrogen phosphate); lubricants (for example, magnesium stearate, talc, or silica, stearic acid, sodium stearyl fumarate, glyceryl behenate, calcium stearate, etc.); disintegrants (for example, potato starch or sodium starch glycolate); or wetting agents (for example, sodium lauryl sulfate), colorants and flavorings, gelatin, sweeteners, natural and synthetic gums (such as acacia gum, tragacanth gum, or alginates), buffering salts, carboxylmethyl cellulose, polyethylene glycol, waxes, etc. For oral administration in liquid form, the pharmaceutical ingredients can be combined with non-toxic, pharmaceutically acceptable inert carriers (for example, ethanol, glycerin, water), anti-sedimentation agents (for example, sorbitol syrup, cellulose derivatives, or hydrogenated edible fats), emulsifiers (for example, lecithin or acacia gum), non-aqueous carriers (for example, almond oil, oil esters, ethanol, or fractionated vegetable oils), preservatives (for example, methyl p-hydroxybenzoate or propyl p-hydroxybenzoate or sorbic acid), etc. Stabilizers such as antioxidants (BHA, BHT, propyl gallate, sodium ascorbate, citric acid) can also be added to stabilize the dosage form.

Tablets containing the active compound can be coated using methods well-known in the field. The compositions of the present disclosure, which include the compound represented by formula I as the active compound, can also be introduced into small beads, microspheres, or microcapsules, for example, constructed with polyglycolic acid/lactic acid (PGLA). Liquid formulations for oral administration can take the forms of e.g. solution, syrup, emulsion, or suspension, or they can be presented as dry products that are reconstituted with water or other suitable excipients before use. Formulations for oral administration can be suitably formulated to provide controlled or delayed release of the active compound.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" contains a small molecule drug linked to a monoclonal antibody through a chemical linker, in which the monoclonal antibody functions as carrier that transports the small molecule drug to its target cells.

As used herein, the terms "reduce," "inhibit," "alleviate," or "decrease" are relative to controls. Those skilled in the art will easily determine the appropriate control for each experiment. For example, a diminished response in subjects or cells treated with the compound is compared to the response in subjects or cells not treated with the compound.

Unless otherwise specified, the raw materials and equipment used in the specific embodiments of the present disclosure are known products that can be obtained by purchasing commercial products.

### Preparation Example 1:

General synthetic method A: formaldehyde (1-3 eq), amine compound (1-2 eq) and dioxane were added to a reaction flask, heated to 50-70°C and reacted for 1 hour under stirring. After cooling to room temperature, 10-hydroxycamptothecin (1 eq) was added, and the mixture was heated to 70-90°C, and continuelly stirred to react for 4h, then cooled, filtered, extracted with ethyl acetate, washed with saturated brine, dried over anhydrous sodium sulfate, and purified by silica gel column chromatography (DCM:MeOH=90:1-10:1) to afford the target compound. Boc-protecting group removal: the Boc-protected compound afforded above was added to a reaction flask and ethyl acetate was added to dissolve the product, then a solution of 4 M HCl in ethyl acetate was added at 0°C, and the mixture was stirred for 2h at room temperature, concentrated, recrystallized to afford the final compound.

General synthetic method B: 10-hydroxycamptothecin (1 eq) and hexamethylenetetramine (1.2-2.0 eq) in TFA were heated to 50 to 70°C and reacted for 12h under stirring in an argon atmosphere. The reaction mixture was concentrated, then H₂O was added, and the mixture was stirred for 1 hour. The pH was adjusted to 8-9 with a saturated aqueous solution of NaHCO₃, and the mixture was extracted with ethyl acetate. The aqueous phase was acidified to pH=4-5 with 2N HCl, extracted with ethyl acetate, dried over Na₂SO₄, purified by silica gel column chromatography, washed with MeOH/DCM (1:50), and concentrated to afford 9-formylcamptothecin product.

9-Formyl-10-hydroxycamptothecin (1 eq) and amine compound (2 eq), methanol/dichloromethane were added to a reaction flask, and the mixture was reacted for 30 minutes at room temperature. Sodium cyanoborohydride was then added, and the reaction was continued with stirring for 6 hours at room temperature, filtered, extracted with ethyl acetate, washed with saturated brine, dried over anhydrous sodium sulfate, concentrated and purified by silica gel column chromatography DCM:MeOH=90:1-10:1 to afford the target product.

Boc-protecting group removal: the same to the above method A, and recrystallization was conducted to afford the final compound.

### Example 1: 9-(1-(4-aminopiperidine))methyl-10-hydroxycamptothecin (1)

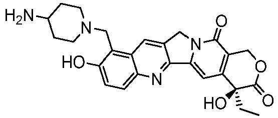

Method A: paraformaldehyde (8.3 mg, 0.274 mmol), 4-N-Boc-aminopiperidine (27 mg, 0.137 mmol) and 1dioxane(10 mL) were heated to 70 °C with stirring and reacted for 2h. After cooling to room temperature, 10-hydroxycamptothecin (25 mg, 0.07mmol) was added, and the temperature was heated to 110°C to continue stirring to react for 6 hours. The mixture was cooled, filtered, extracted with ethyl acetate, dried over anhydrous sodium sulfate, concentrated and purified by column chromatography with the eluent being DCM : MeOH=90:1-10:1, to afford the target compound 9-(4-Boc-aminopiperidine)methyl-10-hydroxycamptothecin (37.1 mg, yield 93.7%); LC/MS (M+H): 577.11 (calculated value : 576.26).

The above 9-(4-Boc-aminopiperidine)methyl-10-hydroxycamptothecin (37.1mg) was dissolved in ethyl acetate (2 mL), a solution of 4 M HCl in ethyl acetate (0.5 mL) was added under stirring at 0°C, and the mixture was stirred for 2h at room temperature. Concentrating was conducted to remove the solvent, and the residue was recrystallized in ethyl acetate/petroleum ether to afford a red solid product compound 9-(1-(4-aminopiperidine))methyl-10-hydroxycamptothecin (30.1 mg, yield 90.9%); ¹H NMR (500 MHz, DMSO-d₆) δ 8.84 (s, 1H), 8.37-8.15 (m, 3H), 8.10 (d, *J*=9.2 Hz, 1H), 7.61 (d, *J*= 9.2 Hz, 1H), 7.28 (s, 1H), 6.35 (s, 3H), 5.42 (s, 2H), 5.26 (s, 2H), 4.48 (s, 2H), 3.28 (d, *J*=11.4 Hz, 1H), 2.95 (s, 2H), 2.03 (d, *J*=12.9 Hz, 2H), 1.87 (dt, *J*=16.2, 7.1 Hz, 2H), 1.83-1.68 (m, 2H), 0.88 (t, *J*=7.3 Hz, 3H); LC/MS (M+H): 476.99 (calculated value : 476.21).

### Example 2: 9-(1-piperazine)methyl-10-hydroxycamptothecin (2)

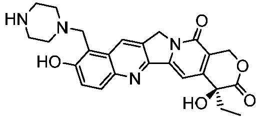

Paraformaldehyde, 1-Boc-piperazine, dioxane, and 10-hydroxycamptothecin were reacted according to Method A to prepare and afford the compound 9-(4-Boc-piperazine-1-yl)methyl-10-hydroxycamptothecin (yield: 92.2%); LC/MS (M+H): 563.05 (calculated value: 562.24).

The above 9-(4-Boc-piperazine-1-yl)methyl-10-hydroxycamptothecin (35.6 mg) was dissolved in ethyl acetate (2 mL), a solution of 4 M HCl in ethyl acetate (0.5 mL) was added under stirring at 0°C. The mixture was stirred for 2h at room temperature, concentrated, and the residue was recrystallized in ethyl acetate/petroleum ether to afford a solid product compound **9-(1-piperazine)methyl-10-hydroxycamptothecin** (26.8 mg, yiel 91.2%); ¹H NMR (500 MHz, DMSO-d₆) δ 11.59 (s, 1H), 9.93 (s, 2H), 9.10 (s, 1H), 8.13 (dt, J=9.2, 3.0 Hz, 1H), 7.76 (d, J=9.2 Hz, 1H), 7.26 (t, *J*=1.8 Hz, 1H), 5.39 (s, 2H), 5.19-5.15 (m, 2H), 4.80 (s, 2H), 3.73 (s, 2H), 1.97 (s, 1H), 1.90 (s, 2H), 1.86 (dd, *J*=10.7, 7.1 Hz, 2H), 0.87 (t, *J*=7.3 Hz, 3H); LC/MS (M+H): 462.97 (calculated value: 462.19).

### Example 3: 9-(1-(4-aminoxypiperidine))methyl-10-hydroxycamptothecin (3)

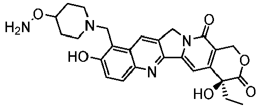

The preparation of 2-(piperidine-4-oxy)isoindoline-1,3-dione: triphenylphosphine (1.93 g, 7.34 mmol) and N-hydroxyphthalimide (1.20 g, 7.34 mmol) were dissolved in THF (10 mL) and stirred at 0 °C. 1-Boc-4-hydroxypyridine (0.500 g, 4.90 mmol) and diethyl azodicarboxylate (3.34 mL, 7.34 mmol) were added, and the mixture was slowly warmed to room temperature and stirred overnight, concentrated to remove the solvent under reduced pressure, and the residue was purified by silica gel column chromatography eluting with petroleum ether/ethyl acetate (90/10 to 50/50) to afford the product 2-(1-Boc-piperidine-4-oxy)isoindoline-1,3-dione (1.8 g, yield 72%); LC/MS (M+H): 347.02 (calculated value: 346.15).

2-(1-Boc-piperidine-4-oxy)isoindoline-1,3-dione (1.8 g, 5.2 mmol) was dissolved in ethyl acetate (5 mL), and a solution of 4 M HCl in ethyl acetate (2 mL) was added. After being stirred for 1 hour at room temperature, the mixture was concentrated to afford 2-(piperidine-4-oxy)isoindoline-1,3-dione hydrochloride (1.31 g, yield 89%); LC/MS (M+H): 247.07 (calculated value: 246.10). Paraformaldehyde, 2-(piperidine-4-oxy)isoindoline-1,3-dione hydrochloride, dioxane, and 10-hydroxycamptothecin were reacted according to Method A to synthesize and afford the compound 9-(2-(piperidine-4-oxy)isoindoline-1,3-dione-1-yl)methyl-10-hydroxycamptothecin (yield 66.5%); LC/MS (M+H): 623.05 (calculated value: 622.21).

9-(2-(Piperidine-4-oxy)isoindoline-1,3-dione-1-yl)methyl-10-hydroxycamptothecin was added to a reaction flask, and ethanol and 85% hydrazine hydrate (1.5 eq) were added. The mixture was stirred for 1 hour at room temperature, concentrated, then converted into hydrochloride by 4M HCl (EA), then recrystallized in ethyl acetate/petroleum ether to afford a red solid compound 9-(1-(4-aminoxypiperidine))methyl-10-hydroxycamptothecin (yield: 85.6%); ¹H NMR (600 MHz, DMSO-d₆) δ 11.50 (s, 1H), 11.01 (s, 2H), 10.19 (s, 1H), 9.07 (s, 1H), 8.16 (d, *J*=9.2 Hz, 1H), 7.74 (dd, *J*=9.2, 6.3 Hz, 1H), 7.28 (s, 1H), 6.55 (s, 1H), 5.42 (s, 2H), 5.24 (s, 2H), 4.74 (s, 2H), 4.41 (d, *J*=43.2 Hz, 1H), 3.30 (s, 2H), 3.06 (dd, *J*=7.3, 4.8 Hz, 1H), 2.19 (s, 3H), 1.88 (dp, *J*=21.6, 7.2 Hz, 2H), 1.55 (s, 1H), 0.89 (t, *J*=7.3 Hz, 3H); LC/MS (M+H): 493.00 (calculated value: 492.20).

### Example 4: 9-(O-ethylhydroxylamine)methyl-10-hydroxycamptothecin (4)

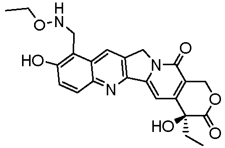

9-Formyl-10-hydroxycamptothecin, O-ethyl hydroxylamine hydrochloride, methanol, and sodium cyanoborohydride were reacted according to Method B to synthesize and afford the compound 9-(O-ethylhydroxylamine)methyl-10-hydroxycamptothecin (yield 56.3%); ¹H NMR (600 MHz, DMSO-d₆) δ 11.37 (s, 1H), 8.87 (s, 1H), 8.69 (s, 2H), 8.15 (d, J=9.1 Hz, 1H), 7.64 (d, J=9.2 Hz, 1H), 7.29 (s, 1H), 6.52 (s, 1H), 5.43 (s, 2H), 5.27 (s, 2H), 4.57 (s, 2H), 3.13 (q, J=7.6, 7.1 Hz, 2H), 1.87 (ddt, J=21.2, 14.5, 7.2 Hz, 2H), 1.27 (t, J=7.2 Hz, 3H), 0.89 (t, J=7.3 Hz, 3H); LC/MS (M+H): 438.08 (calculated value: 437.16).

### Example 5: 9-(1-(4-methoxypiperidine))methyl-10-hydroxycamptothecin (5)

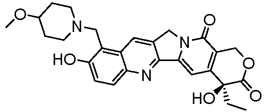

Paraformaldehyde, 4-hydroxymethylpiperidine, dioxane and 10-hydroxycamptothecin were reacted according to Method A to synthesize and afford the compound 9-(4-methoxypiperidine-1-yl))methyl-10-hydroxycamptothecin (yield 81.5%); ¹H NMR (500 MHz, DMSO-d₆) δ 11.39 (s, 1H), 9.30 (s, 1H), 8.94 (s, 1H), 8.19 (d, *J*=9.2 Hz, 1H), 7.64 (d, *J*=9.2 Hz, 1H), 7.29 (s, 1H), 6.52 (s, 1H), 5.43 (s, 2H), 5.29 (s, 2H), 4.72 (s, 2H), 3.36 (s, 5H), 2.13 (s, 1H), 2.04-1.81 (m, 4H), 1.60 (s, 1H), 0.89 (t, *J*=7.3 Hz, 3H); LC/MS (M+H): 492.06 (calculated value : 491.21).

### Example 6: 9-(1-(4-cyclopropylcarbonylpiperazine))methyl-10-hydroxycamptothecin (6)

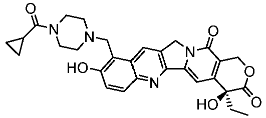

Paraformaldehyde, 1-cyclopropylcarbonylpiperazine, dioxane and 10-hydroxycamptothecin were reacted according to Synthetic Method A to afford the compound 9-(4-cyclopropylcarbonylpiperazine-1-yl)methyl-10-hydroxycamptothecin (yield 91.1%); ¹H NMR (500 MHz, DMSO-d₆) δ 8.74 (d, *J*=9.0 Hz, 1H), 7.96 (t, *J*=8.9 Hz, 1H), 7.45 (dd, *J*=9.5, 4.6 Hz, 1H), 7.25 (d, *J*=3.3 Hz, 1H), 5.41 (s, 2H), 5.20 (d, *J*=11.3 Hz, 2H), 4.02 (d, J=3.8 Hz, 2H), 3.53-3.45 (m, 4H), 3.43-3.39 (m, 1H), 3.37 (t, *J*=6.5 Hz, 1H), 2.58 (s, 2H), 2.02-1.92 (m, 1H), 1.87 (dt, J=16.5, 7.2 Hz, 2H), 1.46 (dd, J=8.5, 6.0 Hz, 1H), 1.35-1.27 (m, 1H), 0.89 (t, J=7.5 Hz, 3H), 0.82-0.60 (m, 4H); LC/MS (M+H): 531.04 (calculated value: 530.22).

### Example 7: 9-(1-(4-acetylpiperazine))methyl-10-hydroxycamptothecin (7)

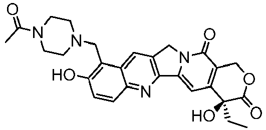

Paraformaldehyde, 1-acetylpiperazine, dioxane and 10-hydroxycamptothecin were reacted according to Method A to synthesize and afford the compound 9-(4-acetylpiperazine-1-yl)methyl-10-hydroxycamptothecin (yield 79.6%); ¹H NMR (500 MHz, DMSO-d₆) δ 11.46 (s, 1H), 9.86 (s, 1H), 8.93 (s, 1H), 8.18 (d, *J*=9.2 Hz, 1H), 7.64 (d, *J*=9.2 Hz, 1H), 7.29 (s, 1H), 6.52 (s, 1H), 5.43 (s, 2H), 5.29 (s, 2H), 4.73 (s, 2H), 3.47 (s, 7H), 2.05 (s, 3H), 1.96-1.78 (m, 2H), 0.89 (t, *J*=7.3 Hz, 3H); LC/MS (M+H): 505.07 (calculated value: 504.20).

### Example 8: 9-(1-(4-aminocarbonylpiperazine))methyl-10-hydroxycamptothecin (8)

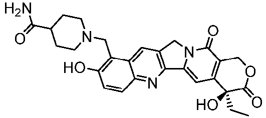

Paraformaldehyde, piperidine-1-carboxamide, dioxane and 10-hydroxycamptothecin were reacted according to Synthetic Method A to afford the compound 9-(4-aminocarbonylpiperazine-1-yl)methyl-10-hydroxycamptothecin (yield 84.2%); ¹H NMR (500 MHz, DMSO-d₆) δ 8.71-8.61 (m, 1H), 7.98-7.88 (m, 1H), 7.41-7.33 (m, 1H), 7.31-7.25 (m, 1H), 7.25-7.21 (m, 1H), 6.77 (s, 1H), 5.49-5.32 (m, 2H), 5.27-5.13 (m, 2H), 3.47 (tdd, *J*=9.7, 6.4, 4.2 Hz, 3H), 3.40 (t, *J*=5.3 Hz, 1H), 3.36 (td, *J*=6.6, 1.9 Hz, 1H), 3.06-2.93 (m, 2H), 2.29-2.13 (m, 2H), 1.86 (dt, *J*=15.0, 7.2 Hz, 2H), 1.76-1.70 (m, 1H), 1.59 (td, J=12.7, 12.3, 3.6 Hz, 2H), 1.51-1.40 (m, 1H), 1.32-1.26 (m, 1H), 0.89-0.85 (m, 3H); LC/MS (M+H): 505.01 (calculated value: 504.20).

### Example 9: 9-(1-(4-methoxycarbonylpiperazine))methyl-10-hydroxycamptothecin (9)

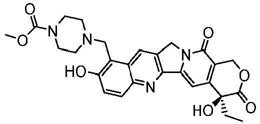

Paraformaldehyde, methyl piperazine-1-carboxylate, dioxane and 10-hydroxycamptothecin were reacted according to Method A to synthesize and afford the compound 9-(4-methoxycarbonylpiperazine-1-yl)methyl-10-hydroxycamptothecin (yield 87.5%); ¹H NMR (500 MHz, DMSO-d₆) δ 11.42 (s, 1H), 9.73 (s, 1H), 8.92 (s, 1H), 8.17 (d, *J*=9.0 Hz, 1H), 7.63 (d, *J*=9.0 Hz, 1H), 7.29 (s, 1H), 6.52 (s, 1H), 5.43 (s, 2H), 5.29 (s, 2H), 4.71 (s, 2H), 4.03 (s, 2H), 3.64 (s, 3H), 3.27 (s, 5H), 1.87 (m, *J*=18.2, 7.2 Hz, 2H), 0.89 (t, *J*=7.3 Hz, 3H); LC/MS (M+H): 521.07 (calculated value : 520.20).

### Example 10: 9-(N-methyl-N-isopropylamino)methyl-10-hydroxycamptothecin (10)

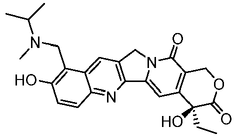

Paraformaldehyde, N-methylpropan-2-amine, dioxane and 10-hydroxycamptothecin were reacted according to Method A to afford the compound 9-(N-methylisopropylamine)methyl-10-hydroxycamptothecin (yield 87.5%); ¹H NMR (500 MHz, DMSO-d₆) δ 8.65 (s, 1H), 7.90 (d, *J*=9.1 Hz, 1H), 7.30 (d, *J*=9.1 Hz, 1H), 7.23 (s, 1H), 5.40 (d, *J*=2.2 Hz, 2H), 5.17 (s, 2H), 4.19 (s, 2H), 3.08 (q, *J*=6.6 Hz, 1H), 2.24 (s, 3H), 1.87 (dq, *J*=14.8, 7.2 Hz, 2H), 1.14 (d, *J*=6.6 Hz, 6H), 0.89 (t, *J*=7.3 Hz, 3H); LC/MS (M+H): 450.04 (calculated value : 449.20).

### Example 11: 9-(N-4-aminobutyl-N-methylamino)methyl-10-hydroxycamptothecin (11)

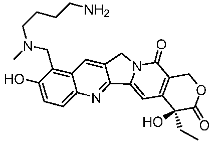

Paraformaldehyde, tert-butyl (4-(methylamino)butyl)-1-carbamate, dioxane and 10-hydroxycamptothecin were reacted according to Method A to synthesize and afford the compound 9-(N-4-Boc-aminobutyl-N-methylamino)methyl-10-hydroxycamptothecin (yield: 82.7%); LC/MS (M+H): 579.07 (calculated value : 578.27).

9-(N-4-Boc-aminobutyl-N-methylamino)methyl-10-hydroxycamptothecin was dissolved in ethyl acetate, stirred at 0°C, then a solution of 4 M HCl in ethyl acetate was added, and the mixture was stirred for 2h at room temperature, concentrated and recrystallized to afford a red solid compound 9-(N-4-aminobutyl-N-methylamino)methyl-10-hydroxycamptothecin (yield 88.3%); ¹H NMR (600 MHz, DMSO-d₆) 8.93-8.84 (m, 1H), 8.73 (s, 1H), 8.19 (ddd, *J*=19.2, 9.2, 3.4 Hz, 1H), 7.90-7.71 (m, 2H), 7.64 (td, *J*=8.9, 8.5, 3.7 Hz, 1H), 7.30 (s, 1H), 6.53 (s, 1H), 5.43 (s, 2H), 5.29 (s, 2H), 4.86-4.63 (m, 1H), 2.90-2.79 (m, 1H), 2.71 (d, *J*=32.2 Hz, 2H), 1.87 (m, *J*=21.4, 14.3, 7.2 Hz, 2H), 1.59(d, J=7.9 Hz, 1H), 1.23 (s, 1H), 0.88(t , *J*=7.3 Hz, 3H); LC/MS (M+H): 479.04 (calculated value : 478.22).

### Example 12: 9-(N-methylhydroxyethylamino)methyl-10-hydroxycamptothecin (12)

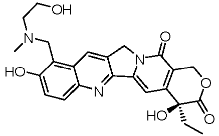

Paraformaldehyde, N-methylethanolamine, dioxane and 10-hydroxycamptothecin were reacted according to Method A to synthesize and afford the compound 9-(N-methylhydroxyethylamino)methyl-10-hydroxycamptothecin (yield 90.7%); ¹H NMR (500 MHz, DMSO-d₆) δ 11.44 (s, 1H), 9.20 (s, 1H), 8.88 (s, 1H), 8.19 (d, *J*=9.2 Hz, 1H), 7.64 (dd, *J*=9.2, 1.9 Hz, 1H), 7.30 (s, 1H), 6.52 (s, 1H), 5.43 (s, 3H), 5.28 (s, 2H), 4.87 (s, 1H), 4.74 (s, 1H), 3.86 (s, 2H), 2.80 (s, 3H), 1.87 (dp, *J*=21.4, 7.2 Hz, 2H), 0.89 (t, *J*=7.3 Hz, 3H); LC/MS (M+H): 452.00 (calculated value : 451.17).

### Example 13: 9-(N-methyl-N-aminoethylamino)methyl-10-hydroxycamptothecin (13)

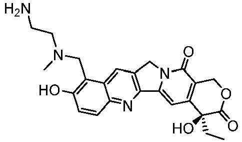

Method B: 9-formyl-10-hydroxycamptothecin (25 mg, 0.064 mmol), tert-butyl (2-(methylamino)ethyl)carbamate (10.2 mg, 0.07 mmol) and methanol (3 mL) were added into a reaction flask, and the mixture was reacted at 20°C for 1h under stirring, then cooled down to 0°C, and sodium cyanoborohydride (4.4 mg, 0.07 mmol) was added, and the mixture was continued to react at 20°C under stirring for 2h, concentrated, and purified by silica gel column chromatography (DCM/MeOH: 90:1-10:1) to afford the compound 9-(N-methyl-N-Bocaminoethylamino)methyl-10-hydroxycamptothecin. (34.1 mg, yield 95.7%); LC/MS (M+H): 551.21 (calculated value : 550.24).

9-(N-Methyl-N-Bocaminoethylamino)methyl-10-hydroxycamptothecin (34.1 mg) was dissolved in ethyl acetate (2 mL), stirred at 0°C, and a solution of 4 M HCl in ethyl acetate (0.5 mL) was added. The mixture was stirred for 2 h at room temperature, concentrated and recrystallised in ethyl acetate/petroleum ether to afford a light red solid compound 9-(N-methyl-N-aminoethylamino)methyl-10-hydroxycamptothecin (27.6 mg yield 87.7%); ¹H NMR (500 MHz, DMSO-d₆) δ 11.65 (s, 1H), 9.77 (s, 1H), 8.89 (s, 1H), 8.17 (d, *J*=9.2 Hz, 1H), 8.14-7.78 (m, 2H), 7.65 (d, *J*=9.1 Hz, 1H), 7.30 (s, 1H), 5.43 (s, 2H), 5.28 (s, 2H), 4.68 (s, 2H), 3.24 (s, 5H), 2.77 (s, 2H), 1.88 (dp, *J*=14.3, 7.1 Hz, 2H), 0.89 (t, J = 7.2 Hz, 3H); LC/MS (M+H): 451.19 (calculated value: 450.19).

### Example 14: 9-(N-methyltetrahydropyran-4-amine)methyl-10-hydroxycamptothecin (14)

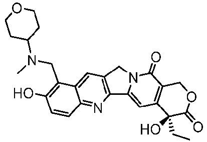

Paraformaldehyde, N-methyltetrahydro-2H-pyran-4-amine, dioxane and 10-hydroxycamptothecin were reacted according to Method A to synthesize and afford the compound 9-(N-methyltetrahydropyran-4-amino)methyl-10-hydroxycamptothecin (yield: 88.7%); ¹H NMR (500 MHz, DMSO-d₆) δ 11.56 (s, 1H), 9.22 (s, 1H), 8.87 (s, 1H), 8.20 (dd, *J*=9.2, 2.3 Hz, 1H), 7.66 (dd, *J*=9.2, 2.0 Hz, 1H), 7.29 (d, *J*=1.1 Hz, 1H), 6.53 (s, 1H), 5.43 (s, 2H), 5.35-5.19 (m, 2H), 4.73 (d, *J*=120.2 Hz, 2H), 4.15-3.98 (m, 2H), 3.71 (s, 1H), 2.67 (s, 3H), 2.19-2.06 (m, 2H), 1.88 (ddq, *J*=21.3, 14.1, 7.1, 6.5 Hz, 4H), 0.89 (t, *J*=7.3 Hz, 3H); LC/MS (M+H): 492.12 (calculated value : 491.21).

### Example 15: 9-(trans-4-hydroxycyclohexylamine)methyl-10-hydroxycamptothecin (15)

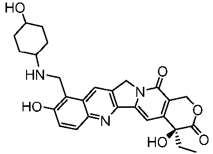

Paraformaldehyde, *trans*-4-aminocyclohexan-1-ol, dioxane and 10-hydroxycamptothecin were reacted according to Method A to synthesize and afford the compound 9-(4-hydroxycyclohexylamine)methyl-10-hydroxycamptothecin (yield 76.9%); ¹H NMR (500 MHz, DMSO-d₆) δ 8.64-8.54 (m, 1H), 7.94-7.82 (m, 1H), 7.34-7.10 (m, 2H), 5.39 (s, 2H), 5.27-5.11 (m, 2H), 4.36 (s, 2H), 4.30-4.12 (m, 4H), 3.41 (dp, *J*=8.5, 4.2 Hz, 2H), 1.98 (d, *J*=10.0 Hz, 2H), 1.85 (tq, *J*=14.5, 8.4, 7.8 Hz, 4H), 1.19 (q, *J*=12.5 Hz, 4H), 0.89 (t, *J*=7.3 Hz, 3H); LC/MS (M+H): 492.09 (calculated value: 491.21).

### Example 16: 9-(N-methyl-trans-4-hydroxycyclohexylamine)methyl-10-hydroxycamptothecin(16)

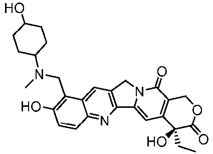

Paraformaldehyde, trans-4-(methylamino)cyclohexan-1-ol, dioxane and 10-hydroxycamptothecin were reacted according to Method A to synthesize and afford the compound 9-(N-methyl-4-hydroxycyclohexylamine)methyl-10-hydroxycamptothecin (yield 82%); ¹H NMR (600 MHz, DMSO-d₆) δ 11.63 (s, 1H), 9.15 (s, 1H), 8.86 (s, 1H), 8.19 (d, *J*=9.1 Hz, 1H), 7.66 (d, *J*=9.2 Hz, 1H), 7.28 (s, 1H), 6.55 (s, 1H), 5.43 (s, 2H), 5.26 (s, 2H), 4.77 (d, *J*=12.7 Hz, 1H), 4.72-4.51 (m, 1H), 3.50-3.39 (m, 2H), 2.65 (s, 3H), 2.26-2.08 (m, 2H), 2.00 (s, 2H), 1.93-1.62 (m, 4H), 1.29 (td, *J*=13.2, 6.6 Hz, 2H), 0.89 (t, *J*=7.4 Hz, 3H); LC/MS (M+H): 506.02 (calculated value : 505.22).

### Example 17: 9-(N-methyl-N-cyclopropylamine)methyl-10-hydroxycamptothecin (17)

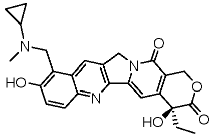

Paraformaldehyde, N-methylcyclopropylamine, dioxane and 10-hydroxycamptothecin were reacted according to Method A to synthesize and afford the compound 9-(N-methyl-N-cyclopropylamine)methyl-10-hydroxycamptothecin (yield 83.9%); ¹H NMR (500 MHz, DMSO-d₆) δ 8.55 (s, 1H), 7.87 (d, *J*=9.1 Hz, 1H), 7.35 (d, *J*=9.2 Hz, 1H), 7.17 (s, 1H), 5.34 (d, J=2.7 Hz, 2H), 5.13 (s, 2H), 4.09 (s, 2H), 3.42 (tdd, *J*=9.9, 6.5, 4.2 Hz, 3H), 3.35 (t, *J*=5.3 Hz, 1H), 3.30 (t, *J*=6.6 Hz, 1H), 2.19 (s, 3H), 1.86 (ddt, *J*=9.8, 7.1, 3.1 Hz, 1H), 1.79 (p, *J*=7.1 Hz, 2H), 1.39 (dd, *J*=8.4, 6.2 Hz, 1H), 1.29-1.19 (m, 1H), 0.87-0.81 (m, 3H); LC/MS (M+H): 448.02 (calculated value: 447.18).

### Example 18: 9-(morpholin-4-amine)methyl-10-hydroxycamptothecin (18)

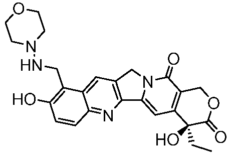

9-Formyl-10-hydroxycamptothecin, 4-aminomorpholine, methanol and sodium cyanoborohydride were reacted according to Method B to synthesize and afford the compound 9-(morpholin-4-amine)methyl-10-hydroxycamptothecin (yield 81.0%); LC/MS (M+H): 479.08 (calculated value: 478.19).

### Example 19: 9-(N-methylethylamine)methyl-10-hydroxycamptothecin (19)

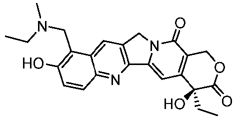

Paraformaldehyde, N-methylethylamine, dioxane and 10-hydroxycamptothecin were reacted according to Method A to synthesize and afford the compound 9-(N-methyl-N-ethylamine)methyl-10-hydroxycamptothecin (yield 77.3%); ¹H NMR (500 MHz, DMSO-d₆) δ 11.45 (s, 1H), 8.88 (s, 1H), 8.18 (d, J=9.2 Hz, 1H), 7.64 (d, J=9.2Hz, 1H), 7.29 (s, 1H), 6.52 (s, 1H), 5.43 (s, 2H), 5.27 (s, 2H), 4.70 (s, 2H), 3.28 (m, J=7.0 Hz, 2H), 2.73 (s, 3H), 1.87 (d, J=21.5, 7.2 Hz, 2H), 1.33 (t, J=7.2 Hz, 3H), 0.89 (t, *J*=7.3 Hz, 3H); LC/MS (M+H): 436.02 (calculated value: 435.18).

### Example 20: 9-(ethylamine)methyl-10-hydroxycamptothecin (20)

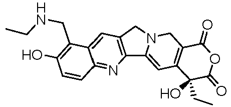

Paraformaldehyde, ethylamine, dioxane and 10-hydroxycamptothecin were reacted according to Method A to synthesize and afford the compound 9-(N-ethylamine)methyl-10-hydroxycamptothecin (yield: 84.0%); ¹H NMR (500 MHz, DMSO-d₆) δ 8.74 (s, 1H), 7.98 (d, J=9.1 Hz, 1H), 7.47 (t, J=9.2 Hz, 1H), 7.25 (s, 1H), 5.41 (s, 2H), 5.23 (s, 2H), 4.42 (s, 2H), 2.85 (dq, J=27.0, 7.3 Hz, 4H), 1.87 (dt, J=15.5, 8.2 Hz, 3H), 1.18 (d, J=7.5 Hz, 3H), 0.88 (t, *J*=7.4 Hz, 3H; LC/MS (M+H): 422.06 (calculated value: 421.16).

### Example 21: 9-(isopropylamine)methyl-10-hydroxycamptothecin (21)

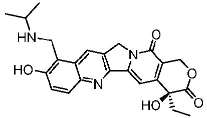

Paraformaldehyde, isopropylamine, dioxane and 10-hydroxycamptothecin were reacted according to Method A to synthesize and afford the compound 9-(isopropylamine)methyl-10-hydroxycamptothecin (yield 79.7%); ¹H NMR (500 MHz, DMSO-d₆) δ 11.45 (s, 1H), 8.83 (s, 1H), 8.60 (s, 2H), 8.14 (d, J=9.2 Hz, 1H), 7.64 (d, J=9.2 Hz, 1H), 7.28 (s, 1H), 6.52 (s, 1H), 5.42 (s, 2H), 5.25 (s, 2H), 4.55 (d, J=6.2 Hz, 2H), 1.87 (dp, J=18.1, 7.1 Hz, 2H), 1.38 (d, J=6.5 Hz, 6H), 0.89 (t, J=7.3 Hz, 3H); LC/MS (M+H): 436.02 (calculated value: 435.18).

### Example 22: 3-(N-morpholine)-1.3-oxazino[5,6][9,10]camptothecin (22)

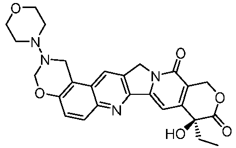

40% Aqueous formaldehyde solution, morpholin-4-amine, dioxane and 10-hydroxycamptothecin were reacted according to Method A to synthesize and afford the compound 3-(N-morpholine)-1.3-oxazino[5,6][9,10]camptothecin (yield 64.3%); LC/MS (M+H): 491.07 (calculated value: 490.19).

### Example 23: 9-(1-methyl-2-Boc-hydrazine)methyl-10-hydroxycamptothecin (23)

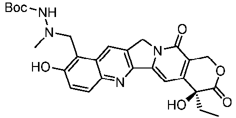

Paraformaldehyde, tert-butyl 2-methylhydrazine-1-carboxylate, dioxane and 10-hydroxycamptothecin were reacted according to Method A to synthesize and afford the compound 1-methyl-2-Boc-hydrazinylmethyl-10-hydroxycamptothecin (yield: 90.7%); ¹H NMR (500 MHz, DMSO-d₆) δ 8.75 (s, 1H), 8.01 (d, J=9.1 Hz, 1H), 7.50 (d, J=9.1 Hz, 1H), 7.26 (s, 1H), 6.44 (d, *J*=56.6 Hz, 2H), 5.41 (s, 3H), 5.24 (s, 2H), 4.93 (s, 2H), 2.41 (s, 3H), 1.93-1.84 (m, 2H), 1.48 (s, 9H), 0.89 (d, *J*=7.3 Hz, 3H); LC/MS (M+H): 523.08 (calculated value: 522.21).

### Example 24: 9-(1-methylhydrazine)methyl-10-hydroxycamptothecin (24)

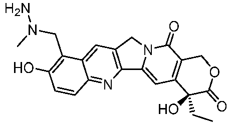

1-Methyl-2-Boc hydrazinylmethyl-10-hydroxycamptothecin (compound **23)** was dissolved in ethyl acetate (2 mL), stirred at 0 °C, and a solution of 4 M HCl in ethyl acetate (0.5 mL) was added. The mixture was stirred for 2 h at room temperature, concentrated, and recrystallized in petroleum ether/ethyl acetate to obtain the solid product 1-methylhydrazinylmethyl-10-hydroxycamptothecin (yield 89.5%); ¹H NMR (600 MHz, DMSO-d₆) δ 10.86 (s, 1H), 9.97 (s, 2H), 8.72 (s, 1H), 8.05 (d, *J*=9.2 Hz, 1H), 7.57 (d, *J*=9.2 Hz, 1H), 7.27 (s, 1H), 6.51 (s, 1H), 5.86 (s, 1H), 5.42 (s, 2H), 5.26 (s, 2H), 4.49 (s, 2H), 2.75 (d, *J*=26.1 Hz, 3H), 1.87 (dq, *J*=10.7, 7.1 Hz, 2H), 0.89 (t, *J*=7.3 Hz, 3H); LC/MS (M+H): 423.08 (calculated value: 422.16).

### Example 25: 9-(1-methyl-3-aminopropylamine)methyl-10-hydroxycamptothecin (25)

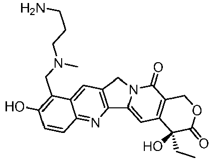

9-Formyl--10-hydroxycamptothecin, tert-butyl (3-(methylamino)propyl)carbamate and sodium cyanoborohydride were reacted according to Method B to synthesize and afford the compound 9-(1-methyl-3-Boc-aminopropylamino)methyl-10-hydroxycamptothecin (yield 90.6%); LC/MS (M+H): 565.17 (calculated value: 564.26).

The above Boc-group protected product was dissolved in ethyl acetate (2 mL), stirred at 0°C, and a solution of 4 M HCl in ethyl acetate (0.5 mL) was added. The mixture was stirred for 2h at room temperature, concentrated to remove the solvent, and the residue was recrystallized in ethyl acetate/petroleum ether to afford a solid product 9-(1-methyl-3-aminopropylamine)methyl-10-hydroxycamptothecin (yield 85.5%); ¹H NMR (500 MHz, DMSO-d₆) δ 8.55 (s, 1H), 7.87 (d, *J*=9.1 Hz, 1H), 7.35 (d, *J*=9.2 Hz, 1H), 7.17 (s, 1H), 5.34 (d, *J*=2.7 Hz, 2H), 5.13 (s, 2H), 4.09 (s, 2H), 3.42 (m, J=9.9, 6.5, 4.2 Hz, 3H), 3.35 (t, J=5.3 Hz, 1H), 3.30 (t, J=6.6 Hz, 1H), 2.19 (s, 3H), 1.86 (m, J=9.8, 7.1, 3.1 Hz, 1H), 1.79 (p, J=7.1 Hz, 2H), 1.39 (dd, J=8.4, 6.2 Hz, 1H), 1.29-1.07 (m, 2H), 0.87-0.81 (m, 3H); LC/MS (M+H): 465.09 (calculated value: 464.21).

### Example 26: 9-(piperidine-1-amine)methyl-10-hydroxycamptothecin (26)

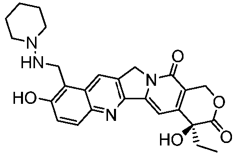

9-Formyl-10-hydroxycamptothecin, 1-aminopiperidine, methanol and sodium cyanoborohydride were reacted according to Method B to synthesize and afford the compound 9-(piperidin-1-amine)methyl-10-hydroxycamptothecin (yield 87.6%); LC/MS (M+H): 477.20 (calculated value: 476.21).

### Example 27: 9-(N-methyl(tetrahydropyranmethyl)amine)methyl-10-hydroxycamptothecin (27)

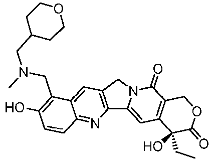

Paraformaldehyde, N-methyl-1-(tetrahydro-2H-pyran-4-yl)methylamine, dioxane and 10-hydroxycamptothecin were reacted according to Method A to synthesize and afford the compound 9-(N-methyl(tetrahydropyranmethyl)amine)methyl-10-hydroxycamptothecin (yield 87%); ¹H NMR (500 MHz, DMSO-d₆) δ 11.75 (s, 1H), 8.88 (d, *J*=3.3 Hz, 1H), 8.18 (dd, *J*=9.3, 3.0 Hz, 1H), 7.65 (dd, *J*=9.4, 3.6 Hz, 1H), 7.29 (d, *J*=2.9 Hz, 1H), 6.54 (s, 1H), 5.42 (d, *J*=3.1 Hz, 2H), 5.26 (s, 2H), 4.76 (s, 2H), 3.88 (d, *J*=11.3 Hz, 2H), 3.34 (d, *J*=11.6 Hz, 2H), 3.20 (s, 2H), 2.73 (d, *J*=3.5 Hz, 3H), 2.25 (ddd, *J*=11.4, 7.6, 3.9 Hz, 1H), 1.88 (ddd, *J*=16.5, 11.9, 7.2 Hz, 2H), 1.76 (d, *J*=12.8 Hz, 2H), 1.26 (q, *J*=12.3 Hz, 2H), 1.08-0.78 (d, *J*=12.8 Hz, 3H); LC/MS (M+H): 506.09 (calculated value: 505.22).

### Example 28: 9-(N-methyl(piperidine-4-methyl)amine)methyl-10-hydroxycamptothecin (28)

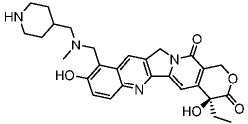

Paraformaldehyde, tert-butyl 4-((methylamino)methyl)piperidine-1-carboxylate, dioxane and 10-hydroxycamptothecin were reacted according to Method A to synthesize and afford the compound 9-(N-methyl(1-Boc-piperidine-4-methyl)amine)methyl-10-hydroxycamptothecin (yield 93.3%): LC/MS (M+H): 605.14 (calculated value: 604.29). The Boc-protected intermediate was dissolved in ethyl acetate (2 mL), stirred at 0°C, and a solution of 4 M HCl in ethyl acetate (0.5 mL) was added. The mixture was stirred for 2h at room temperature, concentrated and the residue was recrystallized to afford a solid product 9-(N-methyl(piperidine-4-methyl)amine)methyl-10-hydroxycamptothecin (yield 88.5%); ¹H NMR (500 MHz, DMSO-d₆) δ 11.65 (s, 1H), 8.89 (s, 1H), 8.19 (dd, J=9.7, 3.8 Hz, 1H), 7.64 (dd, J=9.2, 1.7 Hz, 1H), 7.29 (d, J=2.1 Hz, 1H), 6.52 (s, 1H), 5.42 (s, 2H), 5.28 (d, J=5.8 Hz, 2H), 4.76 (s, 2H), 3.87 (dt, *J*=10.9, 3.4 Hz, 2H), 3.34 ((td, *J*=11.8 2.1 Hz, 2H), 3.19 (s, 2H), 2.73 (s, 3H), 2.24 (ddd, *J*=11.4, 7.6, 4.1 Hz, 1H), 1.94-1.82 (m, 2H), 1.75 (s, 2H), 1.26 (d, *J*=14.3 Hz, 2H), 0.88 (t, *J*=7.3 Hz, 3H); LC/MS (M+H): 505.14 (calculated value: 504.24).

### Example 29: 9-(3-(undeca-5,5-spirocyclo-3,9-diazane))methyl-10-hydroxycamptothecin (29)

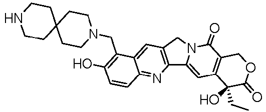

Paraldehyde, tert-butyl 3,9-diazaspiro[5.5]undecane-3-carboxylate, dioxane and 10-hydroxycamptothecin were reacted according to Method A to synthesize and afford the compound 9-(3-(9-Boc-undeca-5,5-spirocyclo-3,9-diazepane))methyl-10-hydroxycamptothecin (yield 92.9%); LC/MS (M+H): 631.18 (Calculated value: 630.31).

The above Boc-intermediate was dissolved in ethyl acetate (2 mL), stirred at 0°C, and a solution of 4 M HCl in ethyl acetate (0.5 mL) was added. The mixture was stirred for 2h at room temperature, concentrated and the residue was recrystallized to afford a solid product -(3-(undeca-5,5-spirocyclo-3,9-diazepane))methyl-10-hydroxycamptothecin (yield 91.1%); ¹H NMR (500 MHz, DMSO-d₆) δ 11.66 (s, 1H), 9.59 (s, 1H), 8.92 (s, 1H), 8.73 (s, 2H), 8.15 (d, J=9.2 Hz, 1H), 7.67 (d, J=9.3 Hz, 1H), 7.28 (s, 1H), 6.52 (s, 1H), 5.42 (s, 2H), 5.23 (s, 2H), 4.75 (s, 2H), 3.69-3.25 (m, 8H), 1.87 (dq, J=14.3, 7.5 Hz, 4H), 1.58 (d, J=82.2 Hz, 4H), 0.90 (t, J=7.3 Hz, 3H); LC/MS (M+H): 531.18 (calculated value: 530.25).

### Example 30: 9-(piperidine-4-ethylamine)methyl-10-hydroxycamptothecin (30)

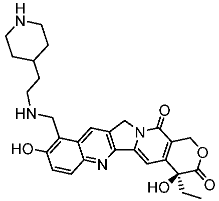

Paraformaldehyde, tert-butyl 4-(2-aminoethyl)piperidine-1-carboxylate, dioxane and 10-hydroxycamptothecin were reacted according to Method A to synthesize and afford the compound 9-(1-Boc-piperidine-4-ethylamine)methyl-10-hydroxycamptothecin (yield 91.1%); LC/MS (M+H): 605.27 (calculated value: 604.29).

The above Boc-intermediate was dissolved in ethyl acetate (2 mL), stirred at 0°C, and a solution of 4 M HCl in ethyl acetate (0.5 mL) was added. The mixture was stirred for 2h at room temperature, concentrated, and the residue was recrystallized in ethyl acetate/petroleum to afford a solid compound 9-(piperidine-4-ethylamine)methyl-10-hydroxycamptothecin (yield 94.5%); ¹H NMR (600 MHz, DMSO-d₆) δ 11.62 (s, 1H), 8.86 (d, *J*=17.7 Hz, 3H), 8.85-8.43 (m, 2H), 8.16 (d, *J*= 9.2 Hz, 1H), 7.67 (d, *J*=9.2 Hz, 1H), 7.29 (s, 1H), 6.53 (s, 1H), 5.43 (s, 2H), 5.27 (s, 2H), 4.65 (s, 2H), 3.20 (t, *J*=6.2 Hz, 6H), 2.80-2.57 (m, 6H), 1.88 (dhept, *J*=21.4, 7.3 Hz, 2H), 0.89 (t, *J*=7.3 Hz, 3H); LC/MS (M+H): 504.87 (calculated value: 504.24).

### Example 31: 9-(L-proline tert-butyl ester)methyl-10-hydroxycamptothecin (31)

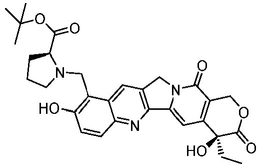

Paraformaldehyde, L-proline tert-butyl ester, dioxane and 10-hydroxycamptothecin were reacted according to Method A to synthesize and afford the compound 9-(L-proline tert-butyl ester)methyl-10-hydroxycamptothecin (yield 90.2%); ¹H NMR (600 MHz, DMSO-d₆) δ 11.55 (s, 1H), 10.08 (s, 1H), 8.89 (s, 1H), 8.15 (d, J=9.0 Hz, 1H), 7.62 (d, J=9.2 Hz, 1H), 7.29 (s, 1H), 6.54 (s, 1H), 5.43 (s, 2H), 5.29 (s, 2H), 4.87 (s, 2H), 4.49 (d, J=43.8 Hz, 1H), 3.19 (s, 2H), 2.46 (s, 1H), 2.04 (d, J=10.0 Hz, 1H), 1.88 (ddt, J=27.3, 14.2, 7.4 Hz, 4H), 1.35 (d, J=4.2 Hz, 9H), 0.89 (t, J=7.3 Hz, 3H); LC/MS (M+H): 548.17 (calculated value: 547.23).

### Example 32: 9-(L-proline)methyl-10-hydroxycamptothecin (32)

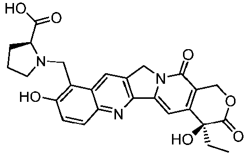

9-(L-Proline tert-butyl ester)methyl-10-hydroxycamptothecin (compound of example 31) was dissolved in dichloromethane (2 mL), stirred at 0°C, and trifluoroacetic acid (2 mL) was added. The mixture was stirred for 2h at room temperature, concentrated, and the residue was recrystallized in acetate/petroleum ether to afford a solid product 9-(L-proline)methyl-10-hydroxycamptothecin (yield 81.4%); ¹H NMR (500 MHz, DMSO-d₆) δ 8.92 (s, 1H), 8.17 (d, J=9.2 Hz, 1H), 7.62 (d, J=9.3 Hz, 1H), 7.30 (d, J=1.5 Hz, 1H), 6.52 (s, 1H), 5.43 (s, 2H), 5.36-5.20 (m, 2H), 4.92 (d, J=13.6 Hz, 1H), 4.82 (d, J=13.7 Hz, 1H), 4.49 (t, J=8.8 Hz, 1H), 3.33 (q, J=10.0, 9.4 Hz, 3H), 2.02 (tq, *J*=12.6, 4.3, 3.2 Hz, 2H), 1.87 (ddd, J=16.6, 14.1, 7.5 Hz, 3H), 0.89 (t, *J*=7.3 Hz, 3H); LC/MS (M+H): 491.99 (calculated value: 491.17).

### Example 33: 9-(1-piperidine-4-methylformate)methyl-10-hydroxycamptothecin (33)

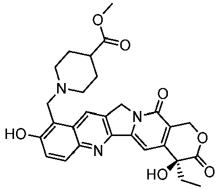

Paraformaldehyde, methyl piperidine-4-carboxylate, dioxane and 10-hydroxycamptothecin were reacted according to Method A to synthesize and afford the compound 9-(1-piperidine-4-methylformate)methyl-10-hydroxycamptothecin (yield 91.3%); ¹H NMR (500 MHz, DMSO-d₆) δ 11.54 (s, 1H), 9.56 (s, 1H), 8.92 (d, *J*=3.7 Hz, 1H), 8.17 (t, *J*=8.5 Hz, 1H), 7.65 (d, *J*=9.2 Hz, 1H), 7.33-7.26 (m, 1H), 5.43 (s, 2H), 5.26 (d, *J*=11.0 Hz, 2H), 4.72 (d, *J*=13.5 Hz, 2H), 3.72 (s, 2H), 3.62 (s, 3H), 3.24 (d, *J*=17.2 Hz, 2H), 2.70 (ddd, *J*=12.3, 8.6, 3.7 Hz, 1H), 2.09-1.96 (m, 2H), 1.86 (qt, *J*=18.5, 10.0 Hz, 4H), 0.89 (t, *J*=7.3 Hz, 3H); LC/MS (M+H): 520.05 (calculated value: 519.20).

### Example 34: 9-(1-piperidine-4-carboxyllic acid)methyl-10-hydroxycamptothecin (34)

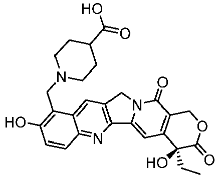

Paraformaldehyde, piperidine-4-carboxyllic acid, dioxane and 10-hydroxycamptothecin were reacted according to Method A to synthesize and afford the compound 9-(1-piperidine-4-carboxyllic acid)methyl-10-hydroxycamptothecin (yield 87.5%); ¹H NMR (500 MHz, DMSO-d₆) δ 12.53 (s, 1H), 11.40 (s, 1H), 9.34 (s, 1H), 8.92 (s, 1H), 8.18 (d, *J*=9.2 Hz, 1H), 7.64 (d, *J*=9.2 Hz, 1H), 7.29 (s, 1H), 6.52 (s, 1H), 5.43 (s, 2H), 5.28 (s, 2H), 4.70 (s, 2H), 3.55 (s, 2H), 3.23 (s, 2H), 2.56 (s, 1H), 2.07-1.98 (m, 2H), 1.87 (dp, *J*=18.3, 7.1 Hz, 2H), 1.78 (s, 1H), 0.89 (t, *J*=7.3 Hz, 3H); LC/MS (M+H): 506.02 (calculated value: 505.18).

### Example 35: 9-(L-alanine tert-butyl ester)methyl-10-hydroxycamptothecin (35)

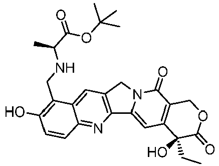

Paraformaldehyde, tert-butylmethyl-L-alanine hydrochloride, dioxane and 10-hydroxycamptothecin were reacted according to Method A to synthesize and afford the compound 9-(L-alanine tert-butyl ester)methyl-10-hydroxycamptothecin (yield 74.8%); ¹H NMR (600 MHz, DMSO-d₆) δ 8.85 (s, 1H), 8.15 (d, *J*=9.1 Hz, 1H), 7.61 (d, *J*=9.2 Hz, 1H), 7.29 (s, 1H), 6.52 (s, 1H), 5.42 (s, 2H), 5.26 (d, *J*=3.6 Hz, 2H), 4.69 (d, *J*=36.8 Hz, 2H), 4.27 (s, 1H), 2.69 (s, 3H), 1.87 (dq, *J*=11.2, 7.0 Hz, 2H), 1.57 (d, *J*=7.0 Hz, 2H), 1.49 (d, *J*=20.5 Hz, 9H), 0.89 (t, *J*=7.3 Hz, 3H); LC/MS (M+H): 522.07 (calculated value: 521.22).

### Example 36: 9-(N-methyl-L-alanine)methyl-10-hydroxycamptothecin (36)

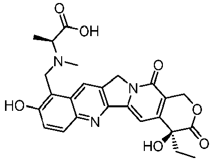

Paraformaldehyde, N-methyl-L-alanine hydrochloride, dioxane and 10-hydroxycamptothecin were reacted according to Method A to synthesize and afford the compound 9-(N-methyl-L-alanine)methyl-10-hydroxycamptothecin (yield 81.4%); ¹H NMR (600 MHz, DMSO-d₆) δ 8.92 (s, 1H), 8.16 (d, *J*=9.1 Hz, 1H), 7.61 (d, *J*=9.2 Hz, 1H), 7.29 (s, 1H), 6.52 (s, 1H), 5.42 (s, 2H), 5.26 (d, *J*=4.4 Hz, 2H), 4.83-4.66 (m, 2H), 4.35 (d, *J*=9.1 Hz, 1H), 2.72 (s, 2H), 1.87 (dp, *J*=21.1, 7.1 Hz, 2H), 1.63 (d, *J*=7.2 Hz, 2H), 0.89 (t, *J*=7.3 Hz, 3H); LC/MS (M+H): 479.92 (calculated value: 479.17).

### Example 37: 9-(tetrahydropyran-4-amine)methyl-10-hydroxycamptothecin (37)

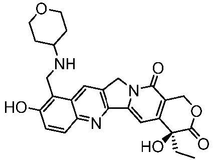

Paraformaldehyde, 4-amino tetrahydropyran, dioxane and 10-hydroxycamptothecin were reacted according to Method A to synthesize and afford the compound 9-(tetrahydropyran-4-amine)methyl-10-hydroxycamptothecin (yield 90.8%); ¹H NMR (500 MHz, DMSO-d₆) δ 11.31 (s, 1H), 8.85 (s, 1H), 8.72 (s, 2H), 8.17 (dt, *J*=9.3, 2.4 Hz, 1H), 7.64 (dt, *J*=9.2, 1.8 Hz, 1H), 7.29 (d, *J*=1.4 Hz, 1H), 6.52 (s, 1H), 5.43 (s, 2H), 5.29 (d, *J*=2.7 Hz, 2H), 4.61 (s, 2H), 3.98 (dd, *J*=10.9, 4.4 Hz, 2H), 2.13 (dd, *J*=11.5, 3.4 Hz, 2H), 1.87 (dq, *J*=18.3, 7.0 Hz, 2H), 1.76-1.62 (m, 2H), 0.89 (t, *J*=7.3 Hz, 3H); LC/MS (M+H): 478.03 (calculated value: 477.19).

### Example 38: 9-(4-Boc-piperazine-1-amine)methyl-10-hydroxycamptothecin (38)

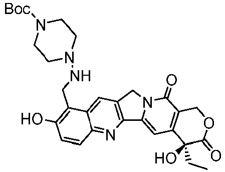

9-Formyl-10-hydroxycamptothecin, tert-butyl 4-aminopiperazine-1-carboxylate, methanol and sodium cyanoborohydride were reacted according to Method B to synthesize and afford the compound 9-(4-Boc-piperazine-1-amine)methyl-10-hydroxycamptothecin (yield: 87.8%); LC/MS (M+H): 578.26 (calculated value: 577.25).

### Example 39: 9-((2-(2-Boc-aminoethoxy)ethyl)amine)methyl)-10-hydroxycamptothecin (39)

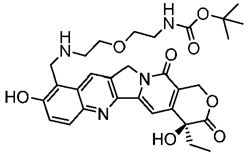

Paraformaldehyde, tert-butyl (2-(2-aminoethoxy)ethyl)carbamate, dioxane and 10-hydroxycamptothecin were reacted according to Method A to synthesize and afford the compound 9-((2-(2-Boc-aminoethoxy)ethyl)amine)methyl)-10-hydroxycamptothecin (yield 81.8%); LC/MS (M+H): 581.23 (calculated value: 580.25).

### Example 40: 3-(N-(2,2,2-trifluoroethyl))-1.3-oxazino[5,6][9,10]camptothecin (40)

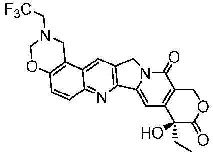

40% Formaldehyde aqueous solution, trifluoroethylamine hydrochloride, dioxane and 10-hydroxycamptothecin were reacted according to Method A to synthesize and afford the compound 3-(N-(2,2,2-trifluoroethyl))-1.3-oxazino[5,6] [9, 10]camptothecin (yield 75.1%); ¹H NMR (500 MHz, DMSO-d₆) δ 8.84 (s, 1H), 8.15 (dd, *J*=9.1, 4.7 Hz, 1H), 7.63 (dd, *J*=9.1, 1.5 Hz, 1H), 6.56 (s, 1H), 5.52 (s, 1H), 4.62 (s, 1H), 3.75 (dd, *J*=11.6, 4.5 Hz, 1H), 3.64 (dd, *J*=11.9, 5.9 Hz, 1H), 1.87 (dt, *J=*16.2, 7.2 Hz, 2H), 1.34 (d, *J*=6.6 Hz, 2H), 0.89 (t, *J*=7.3 Hz, 3H); LC/MS (M+H): 488.01 (calculated value: 487.14).

### Example 41: 9-(trifluoroethylamine)methyl-10-hydroxycamptothecin (41)

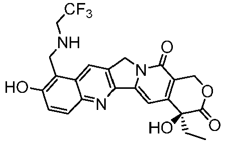

9-Formyl-10-hydroxycamptothecin, trifluoroethylamine, methanol and sodium cyanoborohydride were reacted according to method B to synthesize and afford the compound 9-(trifluoroethylamine)methyl-10-hydroxycamptothecin (yield 71.3%); ¹H NMR (600 MHz, DMSO-d₆) δ 8.77 (s, 1H), 8.07 (d, *J*=10.3 Hz, 1H), 7.56 (d, *J*=9.1 Hz, 1H), 7.44 (t, *J*=7.7 Hz, 1H), 7.33-7.24 (m, 3H), 6.50 (s, 2H), 5.42 (s, 2H), 5.27 (s, 2H), 4.47 (s, 1H), 1.86 (dq, *J*=14.2, 7.1 Hz, 2H), 0.89 (t, *J*=7.3 Hz, 3H); LC/MS (M+H): 476.01 (calculated value: 475.14).

### Example 42: 9-(trans-4-methoxycyclohexylamine)methyl-10-hydroxycamptothecin (42)

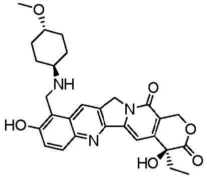

Paraformaldehyde, *trans*-4-methoxycyclohexan-1-amine, dioxane, and 10-hydroxycamptothecin were reacted according to method A to synthesize and afford the compound 9-(*trans*-4-methoxycyclohexylamine)methyl-10-hydroxycamptothecin (yield 82.6%); ¹H NMR (500 MHz, DMSO-d₆) δ 11.37 (s, 1H), 8.83 (s, 1H), 8.63 (s, 2H), 8.15 (d, *J*=9.1 Hz, 1H), 7.64 (d, *J*=9.2 Hz, 1H), 7.29 (s, 1H), 6.52 (s, 1H), 5.76 (s, 1H), 5.43 (s, 2H), 5.27 (s, 2H), 4.58 (s, 2H), 3.26 (s, 3H), 3.17-3.10 (m, 1H), 2.24 (d, *J*=11.0 Hz, 2H), 2.12 (d, *J*=9.5 Hz, 2H), 1.87 (d, *J*=7.8 Hz, 2H), 1.51 (qd, *J*=12.7, 3.3 Hz, 2H), 1.23 (s, 2H), 0.89 (t, *J*=7.3 Hz, 3H); LC/MS (M+H): 506.09 (calculated value: 505.22).

### Example 43: 9-(tetrahydropyran-4-methylamine)methyl-10-hydroxycamptothecin (43)

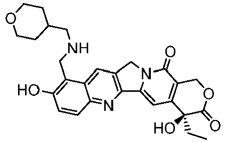

Paraformaldehyde, (tetrahydro-2H-pyran-4-yl)methylamine, dioxane and 10-hydroxycamptothecin were reacted according to method A to synthesize and afford the compound 9-(tetrahydropyran-4-methylamine)methyl-10-hydroxycamptothecin (yield 87.2%); ¹H NMR (500 MHz, DMSO-d₆) δ 11.44 (s, 1H), 8.85 (s, 1H), 8.71 (s, 2H), 8.15 (d, *J*=9.1 Hz, 1H), 7.64 (d, *J*=9.2 Hz, 1H), 7.29 (s, 1H), 6.52 (s, 1H), 5.42 (s, 2H), 5.27 (s, 2H), 4.60 (s, 2H), 3.87 (ddd, *J*=11.5, 4.6, 2.0 Hz, 2H), 3.29 (td, *J*=11.8, 2.1 Hz, 2H), 3.01 (d, *J*=7.1 Hz, 2H), 2.05 (dt, *J*=7.4, 3.6 Hz, 1H), 1.87 (dt, *J*=15.1, 7.1 Hz, 2H), 1.75-1.66 (m, 2H), 1.25 (qd, *J*=12.1, 4.5 Hz, 2H), 0.89 (t, *J*=7.3 Hz, 3H); LC/MS (M+H): 492.06 (calculated value: 491.21).

### Example 44: 9-(N-methylhydroxylamine)methyl-10-hydroxycamptothecin (44)

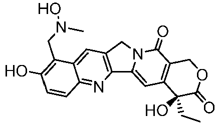

9-Formyl-10-hydroxycamptothecin, N-methylhydroxylamine, methanol and sodium cyanoborohydride were reacted according to method B to synthesize and afford the compound 9-(N-methylhydroxylamine)methyl-10-hydroxycamptothecin (yield 44.1%); 1H NMR (500 MHz, DMSO-d₆) δ 10.88 (s, 1H), 8.98 (s, 1H), 7.91 (d, *J*=9.2 Hz, 1H), 7.60 (d, *J*=9.2 Hz, 1H), 7.18 (s, 1H), 6.46 (s, 1H), 5.38 (s, 2H), 5.13 (s, 2H), 4.71 (s, 1H), 2.51 (s, 3H), 1.83 (hept, *J*=6.7, 6.1 Hz, 2H), 0.85 (t, *J*=7.3 Hz, 3H); LC/MS (M+H): 424.08 (calculated value: 423.14).

### Example 45: 9-(2' -N-Boc-2' -N-methylhydrazine)methyl-10-hydroxycamptothecin (45)

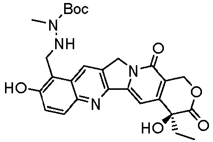

9-Formyl-10-hydroxycamptothecin, tert-butyl 1-methylhydrazine-1-carboxylate, methanol and sodium cyanoborohydride were reacted according to method B to synthesize and afford the compound 9-(2' -N-Boc-2' -N-methylhydrazine)methyl-10-hydroxycamptothecin (yield 74.1%); ¹H NMR (600 MHz, DMSO-d₆) δ 9.36 (s, 1H), 8.71 (s, 1H), 8.10 (d, *J*=10.1 Hz, 2H), 8.04 (s, 1H), 7.55 (d, *J*=9.2 Hz, 1H), 7.28 (s, 1H), 6.51 (s, 1H), 5.42 (s, 2H), 5.27 (s, 2H), 4.24 (t, *J*=5.4 Hz, 1H), 3.47 (s, 3H), 1.87 (dq, *J*=14.2, 7.0 Hz, 2H), 1.56 (s, 9H), 0.91-0.87 (m, 3H); LC/MS (M+H): 523.15 (calculated value: 522.21).

### Example 46: 9-(1-(4-hydroxyacetylpiperazine))methyl-10-hydroxycamptothecin (46)

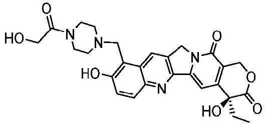

Compound 2 (15 mg, 3.2 mmol) was dissolved in THF (5 mL), then hydroxyacetic acid (5.0 mg, 6.4 mmol) was added, and benzotriazol-1-yloxytris(dimethylamino)phosphonium hexafluorophosphate (BOP, 2.9 mg, 6.4 mmol) was added under stirring. The reaction was stirred overnight at room temperature, and the solvent was removed under reduced pressure. The residue was purified by silica gel column chromatography to afford 9-(1-(4-hydroxyacetylpiperazine))methyl-10-hydroxycamptothecin (11.5 mg, yield 69.3%); ¹H NMR (500 MHz, DMSO-d₆) δ 8.86 (s, 1H), 8.09 (d, *J*=9.2 Hz, 1H), 7.58 (d, *J*=9.2 Hz, 1H), 7.27 (s, 1H), 6.52 (s, 3H), 5.42 (s, 2H), 5.26 (s, 2H), 4.43 (s, 2H), 4.11 (s, 2H), 3.56 (d, J=43.9 Hz, 4H), 3.15-2.89 (m, 4H), 1.87 (dt, J=16.5, 7.2 Hz, 2H), 0.89 (t, J=7.3 Hz, 3H); LC/MS (M+H): 520.99 (calculated value: 520.20).

### Example 47: 9-(N,O-dimethylhydroxylamine)methyl-10-hydroxycamptothecin (47)

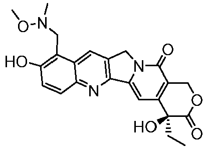

9-Formyl-10-hydroxycamptothecin, N,O-dimethylhydroxylamine hydrochloride, methanol and sodium cyanoborohydride were reacted according to Method B to synthesize and afford the compound 9-(N,O-dimethylhydroxylamine)methyl-10-hydroxycamptothecin (yield 65.3%); ¹H NMR (600 MHz, DMSO-d₆) δ 10.44 (s, 1H), 10.33 (s, 1H), 8.82 (s, 1H), 8.66 (s, 1H), 8.01 (dd, *J*=15.4, 9.2 Hz, 1H), 7.52 (t, *J*=8.5 Hz, 1H), 7.26 (d, J=2.2 Hz, 1H), 6.49 (s, 1H), 5.42 (s, 2H), 5.27 (d, J=4.5 Hz, 2H), 4.87 (s, 1H), 4.28 (s, 1H), 3.33 (d, J=3.4 Hz, 3H), 2.55 (s, 1H), 1.86 (dh, J=21.3, 7.2 Hz, 2H), 1.24 (s, 1H), 0.88 (t, *J*=7.3 Hz, 3H); LC/MS (M+H): 438.11 (calculated value: 437.16).

### Example 48: 9- ((1-methoxypropan-2-yl)amino)methyl-10-hydroxycamptothecin(48)

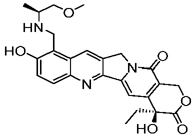

Paraformaldehyde, 1-methoxypropan-2-amine, dioxane and 10-hydroxycamptothecin were reacted according to Method A to synthesize and afford the compound 9- ((1-methoxypropan-2-yl)amino)methyl-10-hydroxycamptothecin (yield 93.2%); ¹H NMR (500 MHz, DMSO-d₆) δ 8.57 (s, 1H), 7.98 (t, J=6.8 Hz, 1H), 7.38 (d, *J*=8.8 Hz, 1H), 7.37-7.29 (m, 1H), 5.48 (d, J=7.4 Hz, 2H), 5.27 (s, 2H), 5.12 (q, J=4.4, 3.9 Hz, 2H), 4.47 (s, 2H), 3.32 (ddd, J=40.3, 11.5, 6.3 Hz, 4H), 3.22 (d, J=8.4 Hz, 3H), 1.93 (q, J=8.2, 7.6 Hz, 2H), 1.22-1.12 (m, 3H), 0.95 (q, *J*=7.6 Hz, 3H); LC/MS (M+H): 466.09 (calculated value: 465.51).

### Example 49: 9-((1-hydroxypropan-2-yl)amino)methyl-10-hydroxycamptothecin (49)

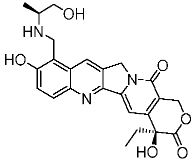

Paraformaldehyde, 1-hydroxypropan-2-amine, dioxane and 10-hydroxycamptothecin were reacted according to Method A to synthesize and afford the compound 9-((1-hydroxypropan-2-yl)amino)methyl-10-hydroxycamptothecin (yield 84.7%); ¹H NMR (600 MHz, DMSO-d₆) δ 11.42 (s, 1H), 8.83 (s, 1H), 8.71 (s, 1H), 8.43 (s, 1H), 8.15 (d, *J*=9.2 Hz, 1H), 7.63 (d, J=9.1 Hz, 1H), 7.29 (s, 1H), 6.54 (s, 1H), 5.50 (s, 1H), 5.43 (s, 2H), 5.27 (s, 2H), 4.61 (s, 2H), 3.75 (dd, *J*=11.7, 4.5 Hz, 1H), 3.63 (dd, J=12.0, 5.8 Hz, 1H), 1.87 (m, J=21.4, 7.2 Hz, 2H), 1.34 (d, J=6.6 Hz, 3H), 0.89 (t, J=7.3 Hz, 3H); LC/MS (M+H): 462.13 (calculated value: 461.48).

### Example 50: 9-(propylamino)methyl-10-hydroxycamptothecin (50)

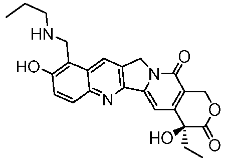

Paraformaldehyde, n-propylamine, dioxane and 10-hydroxycamptothecin were reacted according to Method A to synthesize and afford the compound 9-(propylamino)methyl-10-hydroxycamptothecin (yield 82.6%); ¹H NMR (500 MHz, DMSO-d₆) δ 11.29 (s, 1H), 8.87 (s, 1H), 8.63 (s, 2H), 8.17 (dd, J=9.2, 1.7 Hz, 1H), 7.63 (dd, J=9.2, 2.1 Hz, 1H), 7.29 (s, 1H), 6.52 (s, 1H), 5.43 (s, 2H), 5.29 (d, J=2.8 Hz, 2H), 4.58 (d, J=5.8 Hz, 2H), 3.03 (s, 2H), 1.93-1.80 (m, 2H), 1.71 (q, *J*=7.7 Hz, 2H), 0.94 (t, J=7.4 Hz, 3H), 0.89 (t, *J*=7.3 Hz, 3H); LC/MS (M+H): 436.45 (calculated value: 435.48).

### Example 51: 9-((2-(2-aminoethoxy)ethyl)amine)methyl)-10-hydroxycamptothecin (51)

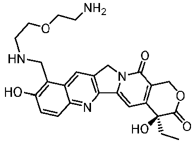

The above 9-((2-(2-Boc-aminoethoxy)ethyl)amine)methyl-10-hydroxycamptothecin **(39)** was dissolved in ethyl acetate (2 mL) and stirred at 0°C, and a solution of 4 M HCl in ethyl acetate (0.5 mL) was added. The mixture was stirred for 2h at room temperature, concentrated, and the residue was recrystallized in ethyl acetate/petroleum ether to afford a solid compound 9-((2-(2-aminoethoxy)ethyl)amine)methyl)-10-hydroxycamptothecin (yield 96.5%); ¹H NMR (500 MHz, DMSO-d₆) δ 11.48 (s, 1H), 9.18 (dq, *J*=12.1, 6.0 Hz, 2H), 9.00 (s, 1H), 8.29 (d, *J*=6.1 Hz, 2H), 8.24 (s, 1H), 7.76 (d, *J*=9.2 Hz, 1H), 7.27 (s, 1H), 5.41 (s, 2H), 5.21 (s, 2H), 4.62 (t, *J*=5.6 Hz, 2H), 3.85-3.65 (m, 6H), 3.25 (p, *J*=5.3 Hz, 2H), 3.03 (tq, *J*=10.7, 5.4 Hz, 2H), 1.88 (dp, *J*=21.2, 7.2 Hz, 2H), 0.90 (t, *J*=7.3 Hz, 3H); LC/MS (M+H): 481.50 (calculated value: 480.52).

### Test Example 1: Inhibitory Activity of Compounds on Cancer Cell Growth

Human esophageal cancer cells OE33, human breast adenocarcinoma cells SK-BR-3 and human gastric cancer cells NCI-N87 were cultured in RPMI1640 (Cellmax) supplemented with 10% fetal bovine serum (Cellmax) respectively. Cells in the exponential growth phase were diluted to a concentration of 1×10⁵cells/mL by culture medium and added to 96-well cell culture plates at 100µL per well, then incubated overnight in a 37°C incubator with 5% CO₂. On the following day, the test compounds and control compounds were diluted to concentrations of 10000 nM, 2000 nM, 400 nM, 80 nM, 16 nM, 3.2 nM, 0.64 nM, and 0.13 nM by culture medium and added to the 96-well cell culture plates at 2µL per well, with three replicate wells for each concentration. Negative control and blank control groups without the addition of compounds were supplemented with 2µL of the dilution liquid per well. After the addition of the compounds, the plates were returned to the 37°C incubator with 5% CO₂ to continue incubation for 72 h. After incubation, the cell culture plates were removed, and the culture medium was aspirated using a pipette. Then, 100µL of culture medium containing 10% CCK-8 was added to each well, and the plates were incubated at 37°C for 3 h. After incubation, the plates were removed, kept in the dark, and placed in ELISA plate. Absorbance was measured at 630nm as the reference wavelength and 450nm as the test wavelength. The IC₅₀ values (Tables 1-3) were calculated using the four-parameter logistic regression method in GraphPad, based on the absorbance values. SN38 (7-ethyl-10-hydroxycamptothecin) and 10CPT (10-hydroxycamptothecin) were used as control compounds. For the IC₅₀ values, wherein "++++" indicates an IC₅₀ < 50 nM; "+++" indicates an IC₅₀ between 50 nM and 100 nM; "++" indicates an IC₅₀ between 100 nM and 500 nM; and "+" indicates an IC₅₀ > 500 nM.

**Table 1: Inhibitory activity of compounds to inhibit the growth of OE33 cells**

| Compound No. | IC₅₀/nM | Compound No. | IC₅₀/nM | Compound No. | IC₅₀/nM |
|---|---|---|---|---|---|
| 1 | ++ | 16 | 0.221 | 31 | ++ |
| 2 | +++ | 17 | +++ | 33 | ++ |
| 3 | ++ | 18 | ++ | 34 | + |
| 5 | +++ | 19 | +++ | 35 | +++ |
| 6 | ++ | 20 | ++ | 36 | ++ |
| 7 | +++ | 21 | ++ | 37 | ++ |
| 8 | ++ | 22 | ++ | 40 | ++ |
| 9 | ++ | 23 | ++++ | 42 | ++ |
| 10 | +++ | 25 | ++ | 43 | ++ |
| 11 | ++ | 26 | ++ | 46 | ++ |
| 12 | ++ | 28 | ++ | 10CPT | ++++ |
| 13 | ++ | 29 | ++ | SN38 | ++++ |
| 14 | ++ | 30 | ++ | | |

**Table 2: Inhibitory activity of compounds to inhibit the growth of SK-BR-3 cells**

| Compound No. | IC₅₀/nM | Compound No. | IC₅₀/nM | Compound No. | IC₅₀/nM |
|---|---|---|---|---|---|
| 1 | +++ | 9 | +++ | 19 | +++ |
| 2 | +++ | 10 | +++ | 21 | ++ |
| 5 | + | 11 | ++ | 10CPT | ++++ |
| 6 | +++ | 12 | ++ | SN38 | ++++ |
| 7 | +++ | 17 | +++ | | |
| 8 | ++ | 18 | +++ | | |

**Table 3: Inhibitory activity of compounds to inhibit the growth of NCI-N87 cells**

| Compound No. | IC₅₀/nM |
|---|---|
| 9 | ++++ |
| 10 | ++++ |
| 17 | ++++ |
| 19 | ++++ |

Results: The compounds provided in the Examples of the present disclosure have a good inhibitory effect on the growth of cancer cells such as esophageal cancer cells OE33, breast adenocarcinoma cells SK-BR-3, and gastric cancer cells NCI-N87. Some compounds, for instance, compounds 10, 17, and 19, exhibited IC₅₀ values less than 100 nM against the growth of these three types of cancer cells, demonstrating a broad-spectrum anticancer activity.

## Claims

1. A compound represented by formula (I) or a pharmaceutically acceptable salt, a stereoisomer, or a prodrug thereof: 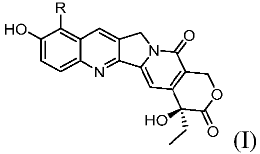 wherein,
R is -CH₂NR₁R₂, wherein, R₁ and R₂ are each independently selected from hydrogen, hydroxyl, C₁-C₆ alkyl, C₁-C₆ alkoxy, C₃-C₆ cycloalkyl, 4 to 8 membered heterocycloalkyl, 4 to 8 membered aryl or -NRₐR_{b}, the C₁-C₆ alkyl, C₁-C₆ alkoxy, C₃-C₆ cycloalkyl, 4 to 8 membered heterocycloalkyl, 4 to 8 membered aryl or -NRₐR_{b} is optionally substituted with substituents selected from halogen, hydroxyl, amino, carboxyl, C₁-C₆ alkoxy, C₁-C₆ alkoxycarbonyl, -O-(C₁-C₃ alkyl)-NRₐR_{b} or 4 to 8 membered heterocycloalkyl, provided that R₁, R₂ are not both hydrogen or methyl; or,
R₁, R₂ and the N atom to which they connect collectively form a 4 to 8 membered cycloalkyl, 4 to 8 membered heterocycloalkyl, 4 to 8 membered aryl or 9 to 12 membered azaspiroalkyl, the 4 to 8 membered cycloalkyl, 4 to 8 membered heterocycloalkyl, 4 to 8 membered aryl or 9 to 12 membered azaspiroalkyl is optionally substituted with substituents selected from hydroxyl, amino, carboxyl, C₁-C₆ alkoxy, C₁-C₆ alkoxycarbonyl, C₁-C₃ alkyl, C₁-C₃ alkoxy, -O-NH₂, -NRₐR_{b} or - C(O)R_{c}; or ,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from C₁-C₆ alkyl, C₁-C₆ alkoxy, C₃-C₆ cycloalkyl, 4 to 8 membered heterocycloalkyl, -NRₐR_{b} or 4 to 8 membered heterocycloalkyl, the C₁-C₆ alkyl, C₁-C₆ alkoxy, C₃-C₆ cycloalkyl, 4 to 8 membered heterocycloalkyl, -NRₐR_{b} or 4 to 8 membered heterocycloalkyl is optionally substituted with substituents selected from halogen, hydroxyl, amino, carboxyl, C₁-C₆ alkoxy or C₁-C₆ alkoxycarbonyl, provided that at least one of R₁, R₂ is H;
Rₐ and R_{b} are each independently selected from H, C₁-C₆ alkyl or C₁-C₆ alkoxycarbonyl;
R_{c} is selected from amino, C₁-C₃ alkyl, C₁-C₃ alkoxy, C₃-C₆ cycloalkyl or hydroxyl-substituted C₁-C₃ alkyl.

2. The compound or pharmaceutically acceptable salt, stereoisomer, or prodrug thereof according to claim 1, wherein, R is -CH₂NR₁R₂, R₁ and R₂ are each independently selected from hydrogen, hydroxyl, C₁-C₆ alkyl, C₁-C₆ alkoxy, C₃-C₆ cycloalkyl, 4 to 8 membered heterocycloalkyl or -NRₐR_{b}, the C₁-C₆ alkyl is optionally substituted with substituents selected from halogen, hydroxyl, amino, carboxyl, C₁-C₆ alkoxycarbonyl, -O-(C₁-C₃ alkyl)-NRₐR_{b} or 4 to 8 membered heterocycloalkyl, the C₃-C₆ cycloalkyl or 4 to 8 membered heterocycloalkyl is optionally substituted with substituents selected from hydroxyl, C₁-C₃ alkoxy or C₁-C₆ alkoxycarbonyl, provided that R₁, R₂ are not both hydrogen or methyl; or,
R₁, R₂ and the N atom to which they connect collectively form a 4 to 8 membered heterocycloalkyl or 9 to 12 membered azaspiroalkyl, the 4 to 8 membered heterocycloalkyl is optionally substituted with substituents selected from hydroxyl, amino, carboxyl, C₁-C₆ alkoxycarbonyl, C₁-C₃ alkyl, C₁-C₃ alkoxy, -O-NH₂ or -C(O)R_{c}; or,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from halogen-substituted C₁-C₆ alkyl, C₃-C₆ cycloalkyl or 4 to 8 membered heterocycloalkyl, provided that at least one of R₁, R₂ is H;
preferably, Rₐ and R_{b} are each independently selected from H, methyl or tert-butoxycarbonyl;
preferably, R_{c} is selected from amino, C₁-C₃ alkyl, C₁-C₃ alkoxy, C₃-C₆ cycloalkyl or hydroxyl-substituted C₁-C₃ alkyl.

3. The compound or pharmaceutically acceptable salt, stereoisomer, or prodrug thereof according to claim 1 or 2, wherein, R is -CH₂NHR₁, R₁ is selected from hydrogen, hydroxyl, C₁-C₆ alkyl, C₁-C₆ alkoxy, C₃-C₆ cycloalkyl, 4 to 8 membered heterocycloalkyl or -NRₐR_{b}, the C₁-C₆ alkyl is optionally substituted with substituents selected from halogen, hydroxyl, amino, carboxyl, C₁-C₆ alkoxycarbonyl, -O-(C₁-C₃ alkyl)-NRₐR_{b} or 4 to 8 membered heterocycloalkyl, the C₃-C₆ cycloalkyl or 4 to 8 membered heterocycloalkyl is optionally substituted with substituents selected from hydroxyl, C₁-C₃ alkoxy or C₁-C₆ alkoxycarbonyl, provided that R₁ is not hydrogen; or ,
R, with the adjacent hydroxyl and the C atom on the benzene ring to which it connects collectively form a 4 to 8 membered heterocycloalkyl, the 4 to 8 membered heterocycloalkyl is optionally substituted with substituents selected from halogen-substituted C₁-C₆ alkyl, C₃-C₆ cycloalkyl or 4 to 8 membered heterocycloalkyl;
preferably, Rₐ and R_{b} are each independently selected from H, methyl or tert-butoxycarbonyl.

4. The compound or pharmaceutically acceptable salt, stereoisomer, or prodrug thereof according to claim 1 or 2, wherein, R₁ and R₂ are each independently selected from hydrogen, hydroxyl, amino, methyl, ethyl, isopropyl, methoxy, ethoxy, trifluoroethyl, cyclopropyl, - (CH₂)₂OH, -(CH₂)₂-₄NH₂, -CH(CH₃)C(O)OH, -CH(CH₃)CH₂OCH₃, -CH(CH₃)CH₂OH, - (CH₂)₂CH₃, -NHBoc, -N(CH₃)Boc, -CH(CH₃)Boc, -(CH₂)₂O(CH₂)₂NH₂, -(CH₂)₂O(CH₂)₂NHBoc, 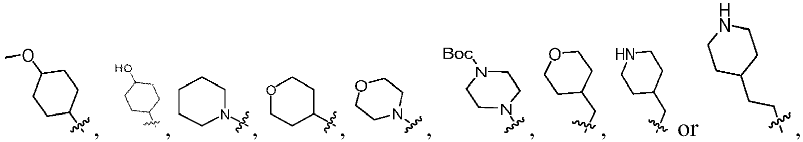 provided that R₁, R₂ are not both hydrogen or methyl; or,
R₁, R₂ and the N atom to which they connect collectively form pyrrolidine, piperidine, piperazine or 3,9-diazaspiroundecane, the pyrrolidine, piperidine, piperazine or 3,9-diazaspiroundecane is optionally substituted with substituents selected hydroxyl, amino, methyl, methoxy, carboxyl, tert-butoxycarbonyl, -O-NH₂ or -C(O)R_{c}; or,
R, with the adjacent hydroxyl and the C atom on the benzene ring to which it connects collectively form 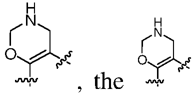 is optionally substituted with substituents selected from trifluoromethyl, cyclopropyl or morpholine-4-yl, provided that at least one of R₁, R₂ is H;
R_{c} is selected from amino, methyl, methoxy, hydroxyethyl or cyclopropyl;
preferably, R is -CH₂NHR₁, R₁ is selected from hydrogen, hydroxyl, amino, methyl, ethyl, isopropyl, methoxy, ethoxy, trifluoroethyl, cyclopropyl, -(CH₂)₂OH, -(CH₂)₂-₄NH₂, - CH(CH₃)COOH, -CH(CH₃)CH₂OCH₃, -CH(CH₃)CH₂OH, -(CH₂)₂CH₃, -NHBoc, N(CH₃)Boc, - CH(CH₃)Boc, -(CH₂)₂O(CH₂)₂NHBoc, 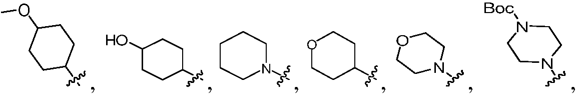 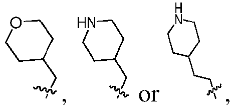 provided that R₁ is not hydrogen; or,
R, with the adjacent hydroxyl and the C atom on the benzene ring to which it connects collectively form 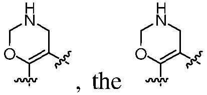 is optionally substituted with substituents selected from trifluoromethyl, cyclopropyl or morpholine-4-yl;
preferably, R is selected from the following structure:

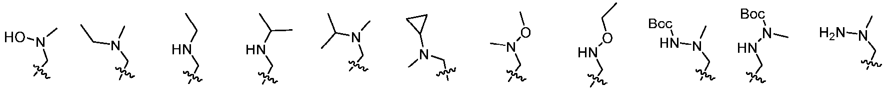

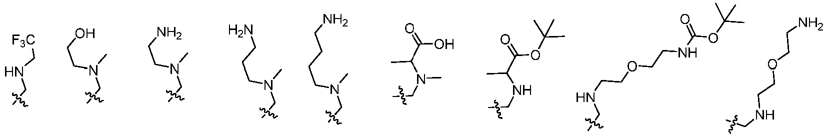

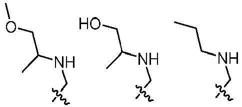

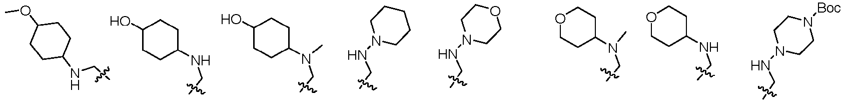

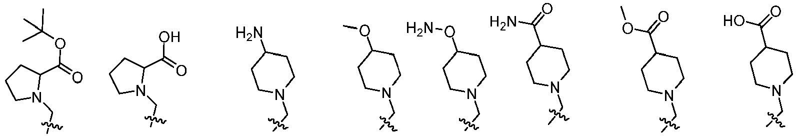

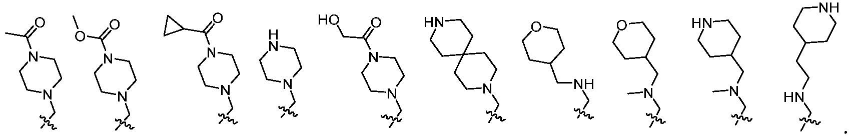

5. The compound or pharmaceutically acceptable salt, stereoisomer, or prodrug thereof according to any one of claims 1 to 4, the compound is the compound represented by formula (II): 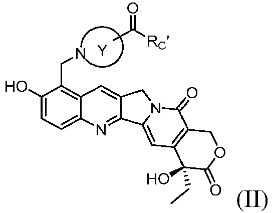 wherein,
ring Y is selected from 4 to 8 membered nitrogen-containing saturated ring, 4 to 8 membered nitrogen-containing aromatic ring or 9 to 12 membered nitrogen-containing saturated spiral ring, the 4 to 8 membered nitrogen-containing saturated ring, 4 to 8 membered nitrogen-containing aromatic ring or 9 to 12 membered nitrogen-containing saturated spiral ring is optionally further substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl;
preferably, ring Y is selected from 4 to 8 membered nitrogen-containing saturated ring or 9 to 12 membered nitrogen-containing saturated spiral ring, the 4 to 8 membered nitrogen-containing saturated ring is optionally substituted with substituents selected from hydroxyl, amino, C₁-C₆ alkoxycarbonyl, C₁-C₃ alkyl, C₁-C₃ alkoxy, -O-NH₂ or -C(O)R_{c}; preferably, the C₁-C₆ alkoxycarbonyl is tert-butoxycarbonyl;
R_{c}' is selected from hydroxyl, amino, C₁-C₃ alkyl, C₁-C₃ alkoxy, C₃-C₆ cycloalkyl or hydroxyl-substituted C₁-C₃ alkyl;
preferably, R_{c}' is selected from hydroxyl, amino, methyl, methoxy, hydroxymethyl, tert-butoxy or cyclopropyl;
preferably, the compound represented by formula (II) is the compound represented by formula (IIa), formula (IIb) or formula (IIc): 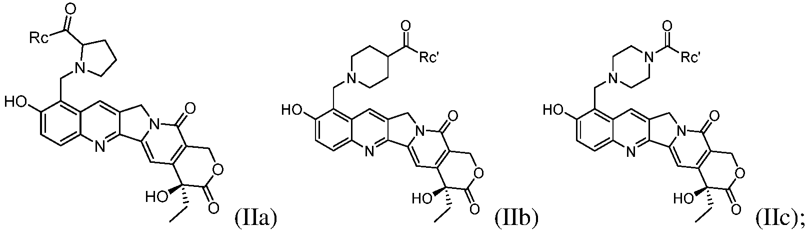
wherein, R_{c}' is selected from hydroxyl, amino, C₁-C₃ alkyl, C₁-C₃ alkoxy, C₃-C₆ cycloalkyl or hydroxyl-substituted C₁-C₃ alkyl;
preferably, Rc' is selected from hydroxyl, amino, methyl, methoxy, hydroxymethyl, tert-butoxy or cyclopropyl.

6. The compound or pharmaceutically acceptable salt, stereoisomer, or prodrug thereof according to any one of claims 1 to 5, the compound is selected from the following compound:
| | | |
|---|---|---|
| 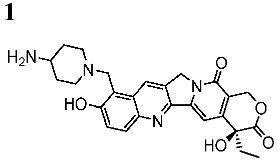 | 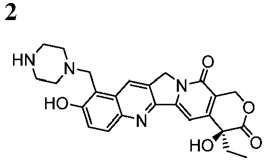 | 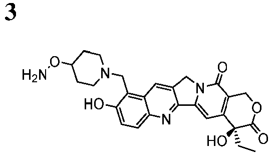 |
| 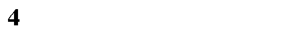 | 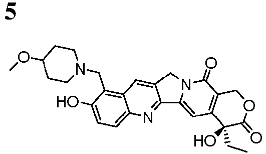 | 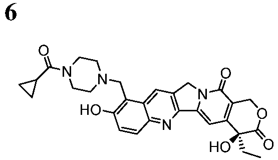 |
| | | |
|---|---|---|
| 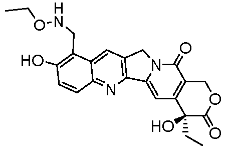 | | |
| 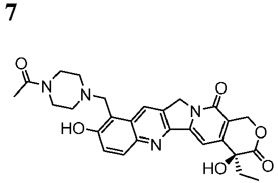 | 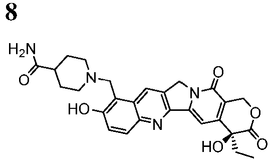 | 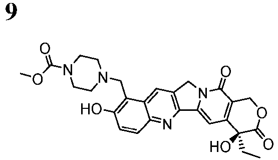 |
| 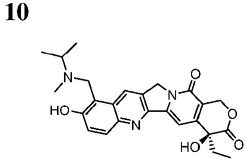 | 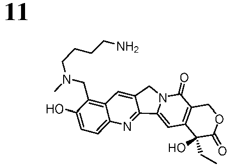 | 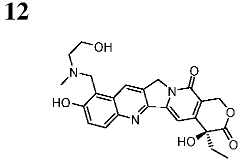 |
| 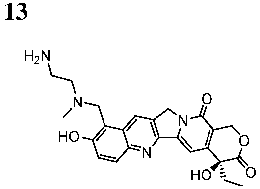 | 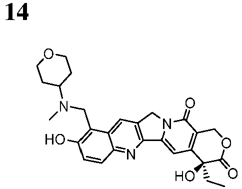 | 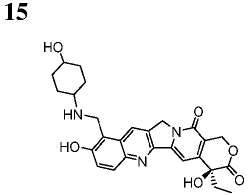 |
| 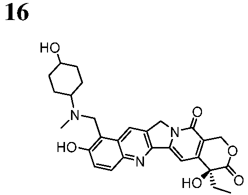 | 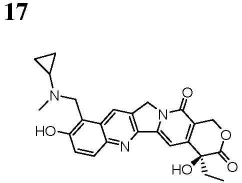 | 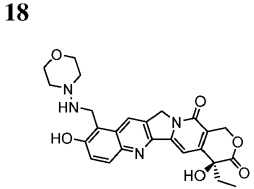 |
| 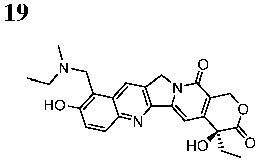 | 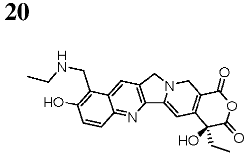 | 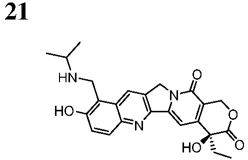 |
| 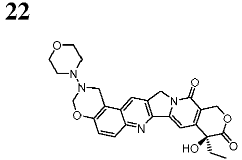 | 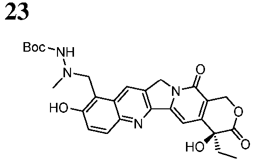 | 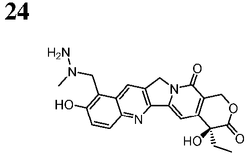 |
| 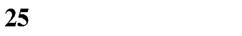 | 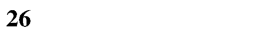 | 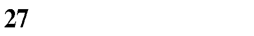 |
| | | |
|---|---|---|
| 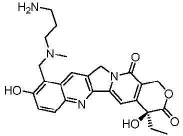 | 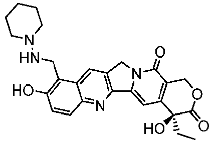 | 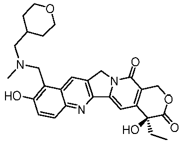 |
| 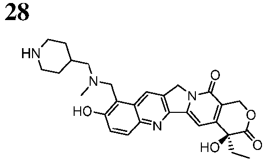 | 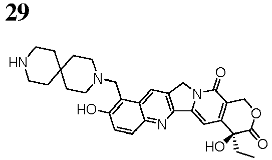 | 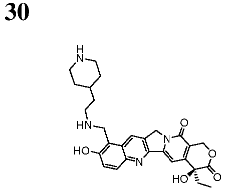 |
| 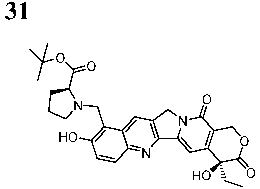 | 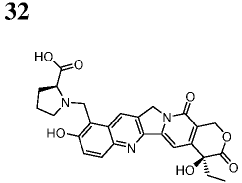 | 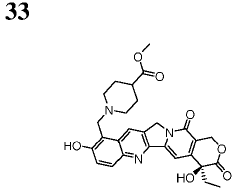 |
| 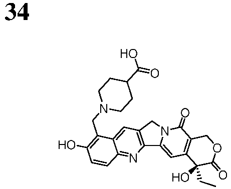 | 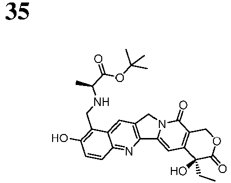 | 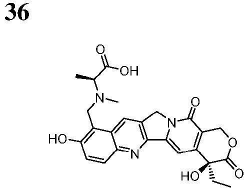 |
| 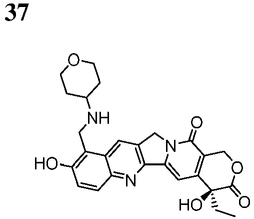 | 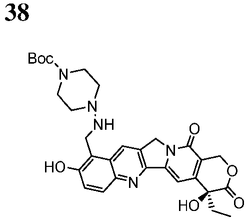 | 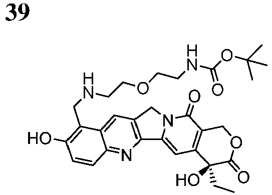 |
| 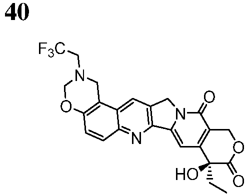 | 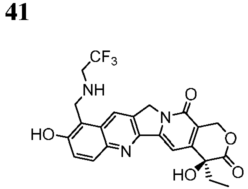 | 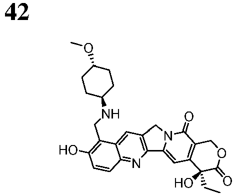 |
| 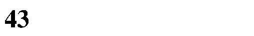 | 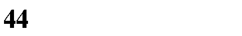 | 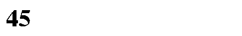 |
| | | |
|---|---|---|
| 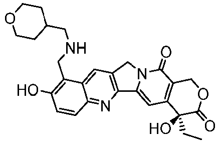 | 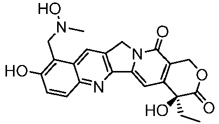 | 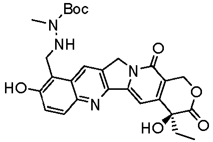 |
| 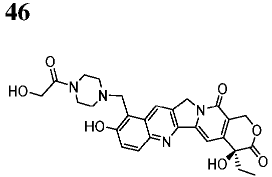 | 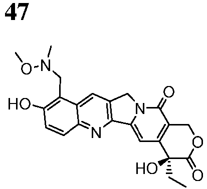 | 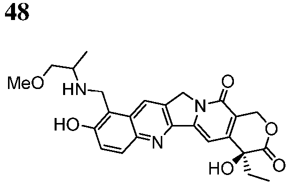 |
| 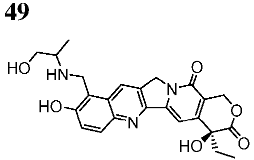 | 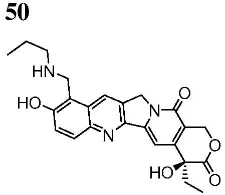 | 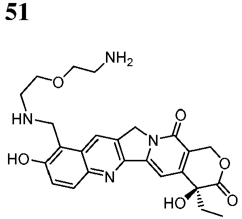 |

7. A method for preparing the compound according to any one of claims 1-6, **characterized by** comprising steps selected from the following reaction scheme: 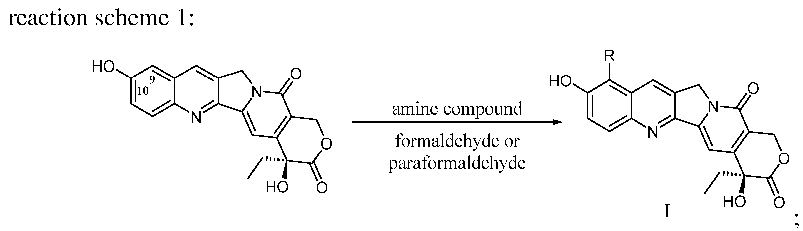
9-position Mannich reaction of 10-hydroxycamptothecin, formaldehyde or paraformaldehyde, and amine compound is carried out to afford the compound represented by formula (I);
wherein, the amine compound is NHR₁R₂, and R, R₁ and R₂ are each defined the same as any one of claims 1-4; 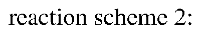 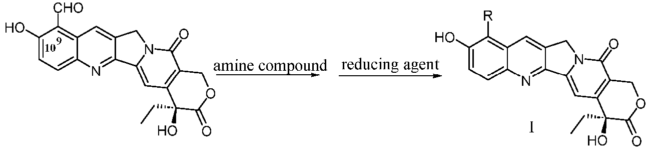
9-formyl-10-hydroxycamptothecin, amine compound and sodium cyanoborohydride are subjected to reductive amination reaction to afford the compound represented by formula (I);
wherein, the amine compound is NHR₁R₂, and R, R₁ and R₂ are each defined the same as any one of claims 1-4;
preferably, the 9-formyl-10-hydroxycamptothecin is afforded by the reaction of 10-hydroxycamptothecin and hexamethylenetetramine; preferably, in the Mannich reaction according to reaction scheme 1, the amount of paraformaldehyde or formaldehyde is 1.0 to 5.0 molar equivalents; preferably, is 1.0 to 3.0 molar equivalents; more preferably, is 1.2 to 2.2 molar equivalents;
preferably, in reaction scheme 1, the amount of amine compound is 1.0 to 5.0 molar equivalents; preferably, is 1.0 to 2.0 molar equivalents; more preferably, is 1.2 to 1.6 molar equivalents;
preferably, in the reductive amination reaction according to reaction scheme 2, the reducing agent is sodium cyanoborohydride;
preferably, in reaction scheme 2, the amount of amine compound is 1.0 to 3.0 molar equivalents; more preferably, is 1.0 to 1.2 molar equivalents;
preferably, the amount of sodium cyanoborohydride is 1.0 to 3.0 molar equivalents; more preferably, is 1.0 to 2.0 molar equivalents; more preferably, is 1.2 to 1.5 molar equivalents.

8. An antibody-drug conjugate, comprising a small molecule drug, a linker and an antibody, **characterized in that**, the compound or pharmaceutically acceptable salt, stereoisomer, or prodrug thereof according to any one of claims 1-6 is the small molecule drug.

9. A pharmaceutical composition, **characterized by** comprising the compound or pharmaceutically acceptable salt, stereoisomer, or prodrug thereof according to any one of claims 1-6, or the antibody-drug conjugate according to claim 8.

10. Use of the compound or pharmaceutically acceptable salt, stereoisomer, or prodrug thereof according to any one of claims 1-6, the antibody-drug conjugate according to claim 8, or the pharmaceutical composition according to claim 9 in the manufacture of a medicament for treating a cancer;
preferably, the cancer includes gastric cancer, esophageal cancer, cardia cancer, breast cancer, ovarian cancer, colon cancer, rectal cancer, primary liver cancer, acute and chronic granulocytic leukemia, choroidal epithelial cancer, lung cancer, bladder cancer, intestinal cancer and small cell lung cancer; more preferably, the cancers are esophageal cancer, breast cancer and gastric cancer.

11. A method of treating a cancer comprising the step of administering to a patient in need thereof the compound or pharmaceutically acceptable salt, stereoisomer, or prodrug thereof according to any one of claims 1-6, or the antibody-drug conjugate according to claim 8, or the pharmaceutical composition according to claim 9.
